# EUROPEAN PATENT APPLICATION

(11) **EP 4 740 953 A1**
(43) Date of publication of application: **13.05.2026**
(21) Application number: 24211838.8
(22) Date of filing: 08.11.2024
(51) Int. Cl.: A61K 33/00, A61J 1/00, A61K 9/00, A61P 11/02, A61P 25/00, A61P 25/06, A61P 25/08, A61P 29/02

(54) **PHARMACEUTICAL COMPOSITION COMPRISING CO2 FOR USE IN THE TREATMENT OF CONDITIONS ASSOCIATED WITH HEADACHE**

(71) Applicant: Migrainy UG (haftungsbeschränkt), 82211 Herrsching (DE)
(72) Inventor: SEIMETZ, Diane, 80636 München (DE); BREMER, Chris-Carol, 30926 Seelze (DE); LOHRMANN, Marc, 82211 Herrsching (DE); MEHLER, Thomas, 82418 Murnau (DE); KLEINERT, Regina, 52074 Aachen (DE)
(74) Representative: DTS Patent- und Rechtsanwälte PartmbB

(57) **Abstract**

The present invention relates to a pressurized pharmaceutical composition comprising carbon dioxide (CO₂) for use in the treatment or prevention of a disorder or condition associated with headache or a disorder or condition involving the trigeminal nervous system in a subject. Furthermore, the present disclosure relates to a device useful for the intranasal application of such compositions. Furthermore, the present invention relates to kits comprising such compositions for use as well as such a device.

## Description

### TECHNICAL FIELD

The present invention relates to a pressurized pharmaceutical composition comprising carbon dioxide (CO₂) for use in the treatment or prevention of a disorder or condition associated with headache or a disorder or condition involving the trigeminal nervous system in a subject. Furthermore, the present disclosure relates to a device useful for the intranasal application of such compositions. Furthermore, the present invention relates to kits comprising such compositions for use as well as such a device.

In general, the present disclosure relates to pressurized compositions comprising carbon dioxide for use in the treatment or prevention of conditions or disorders associated with headache such as migraine. More particularly, this invention is concerned with alleviating migraine and other headache-associated disorders by administering pressurized compositions comprising carbon dioxide intranasally. The invention further pertains to pressurized compositions comprising carbon dioxide for use in the treatment or prevention of conditions involving the trigeminal nerve system, CGRP-involving reactions, pH shift, temporomandibular joint disorder, post-traumatic headache, and/or secondary headache.

### BACKGROUND

Migraine is a type of headache characterized by recurrent attacks of moderate to severe throbbing and pulsating pain on one or both sides of the head. The pain is, amongst others, caused by the activation of nerve fibers within the wall of brain blood vessels traveling inside the meninges (three layers of membranes protecting the brain and spinal cord). Untreated attacks last from four to 72 hours. Other common symptoms include increased sensitivity to light, noise, and odors; nausea; and vomiting.

The two major types of migraine are (i) migraine with aura, previously called classic migraine, which includes visual disturbances and other neurological symptoms that appear about 10 to 60 minutes before the actual headache and usually last no more than an hour; and (ii) migraine without aura, or common migraine, which is the more frequent form of migraine.

Examples of triggers of migraine are: stress related, e.g., anxiety, anger, worry, excitement, shock, depression, overexertion, changes of routine and changes of climate, food-related, e.g., chocolate, cheese and other dairy products, red wine, fried food and citrus fruits, sensory-related, e.g., bright lights or glare, loud noises and intense or penetrating smells, hormonal changes, menstruation and contraceptive drugs.

Migraine affects approximately 8% of the global population. Migraine also most commonly occurs at productive age, in which the frequency of migraine headache days correlate with increased disability, leading to a decreased quality of life involving negative psychological impacts on performances within social and familial contexts. Moreover, the disease represents a significant burden to the health care system.

The pathophysiology of migraine remains poorly understood. However, some specific vasoactive substances and neurotransmitters probably play a role in the mechanism of neurovascular and cortical spreading depression. Calcitonin gene-related peptide (CGRP), neurokinin A, nitric oxide, and substance P are released with perivascular nerve activity. They likely interact with the blood vessel wall, causing dilation, protein extravasation, and sterile inflammation. The stimulation of the trigeminocervical complex by these chemical substances is relayed to the thalamus and cortex, both of which register pain.

Known drugs for the treatment of migraine include acetaminophen, nonsteroidal anti- inflammatory drugs (NSAIDs), triptans, antiemetics, ergot alkaloids, and combination analgesics. Acetaminophen and nonsteroidal anti-inflammatory drugs are first-line treatments for mild to moderate migraines, whereas triptans are first-line treatments for moderate to severe migraines. Although triptans are effective, they may not be (sufficiently) effective in all patients. Other medications such as dihydroergotamine and antiemetics are recommended for use as second- or third-line therapy for select patients or for those with refractory migraine.

Calcitonin gene-related peptide (CGRP) antagonists are a class of medications that act as antagonists of the CGRP receptor or ligand. They can be divided into monoclonal antibodies and non-peptide small molecules, also known as gepants. CGRP antagonists were the first oral agents specifically designed to prevent migraines. Some patients, however, respond only poorly or not at all to CGRP antagonists.

Administration of carbon dioxide has also been proposed as treatment option for migraine, see, e.g., US 2023/0166061 A1, WO 2001/064280 A1, and US 7,017,573 B1.

C. Vause et al. report in Headache. 2007; 47(10): 1385-1397 on the effect of carbon dioxide on calcitonin gene-related peptide secretion from trigeminal neurons. The authors report that CO₂ treatment under isohydric conditions resulted in a decrease in intracellular pH and inhibition of the KCl- and capsaicin mediated increases in intracellular calcium.

Management of migraine is complicated by the lack of a single therapy which is effective in all patients with the same migraine type and by the need to select either an abortive or prophylactic method of treatment for these migraines. Another important consideration is that the more effective antimigraine agents in current use, e.g., the ergots, methysergide, produce severe use-limiting side effects with long term usage.

Thus, there is a need for a safe and effective treatment of acute migraine pain and related disorders which can be used either prophylactically or to alleviate a migraine attack.

It is therefore an object of the present invention to provide for compositions for use in the prevention or treatment of such disorders that can be provided and administered in a fast, safe, reliable and/or reproduceable way. Furthermore, it is an object of the present invention to provide for compositions for use in the prevention or treatment of such disorders that provide for the reduction of unwanted side-effects.

Further objects of the invention may become apparent in view of the present disclosure.

### SUMMARY OF THE INVENTION

In a first aspect, the present invention relates to a pressurized composition comprising carbon dioxide (CO₂) for use
- in the treatment or prevention of a disorder or condition associated with headache, or a disorder or condition involving the trigeminal nervous system in a subject, or
- in the treatment or prevention of allergic rhinitis, rhinosinusitis, trigeminal neuralgia, posttraumatic headache, temporomandibular joint disorder epilepsy or seizures in a subject,

wherein the composition comprising carbon dioxide is administered to the nasal cavity of the subject by intranasal administration in the form of a gaseous composition comprising carbon dioxide, and
wherein the gaseous composition comprising carbon dioxide is administered to the nasal cavity of the subject via both nostrils of the subject.

In a second aspect, the present invention provides for an applicator device for the intranasal administration of a pressurized gaseous composition comprising carbon dioxide to a subject, preferably for the intranasal administration of a composition comprising carbon dioxide for use according to the first aspect of the invention,
wherein the application device is adapted for the administration or introduction of the gaseous composition comprising carbon dioxide into the nasal cavity of the subject by intranasal administration via both nostrils of the subject, preferably via simultaneous administration via both nostrils of the subject.

In a third aspect, the present invention provides a kit comprising:
- a pressurized composition comprising carbon dioxide; and
- an applicator device adapted for the intranasal administration of the pressurized gaseous composition comprising carbon dioxide to the nasal cavity of a subject via both nostrils of a subject, preferably an administration device according to the second aspect of the invention.

In a fourth aspect, the present invention provides for a method for the preparation of a gaseous composition comprising carbon dioxide (suitable for the administration into the nasal cavity of a subject), the method comprising the steps:
- providing a pressurized composition comprising carbon dioxide; and
- at least partly evaporating the pressurized composition comprising carbon dioxide in pressurized form to provide a gaseous composition comprising carbon dioxide (in less pressurized form).

### BRIEF DESCRIPTION OF THE DRAWINGS

Fig. 1 shows a schematic, cross-sectional depiction of an applicator device as described in connection with the pressurized compositions comprising carbon dioxide for use according to the first aspect of the invention.
Fig. 2 depicts an embodiment of the applicator device as shown in Fig. 1 in which the control unit is fluidically connected to the (inner volume of) pre-conditioning chamber of pre-conditioning unit via a dosage regulator channel.
Fig. 3 shows the results of extracellular pH-measurements as described in Example 2.2.
Fig. 4a and 4b depict the results of intracellular pH-measurements as described in Example 2.3.
Fig. 5a and 5b show the observed changes in Hsp70 expression in trigeminal ganglion neuronal cells after exposure to a gaseous composition comprising CO₂ as described in Example 2.4.
Fig 6a and 6b summarize the changes in the intracellular level of calcium caused by the administration of CO₂ for 20 s to trigeminal ganglion neuronal cells as described in Example 2.5.

### DETAILED DESCRIPTION OF THE INVENTION

The following terms or expressions as used herein should normally be interpreted as outlined in this section, unless defined otherwise by the present description or unless the specific context indicates or requires otherwise:
The terms "consist of", "consists of" or "consisting of" as used herein are so-called closed language meaning that only the mentioned components are present. The terms "comprise", "comprises" or "comprising" as used herein are so-called open language, meaning that one or more further components may or may not also be present.

The term "composition" or "pharmaceutical composition", in the context of the present invention, is a preparation comprising a pharmaceutically or physiologically active ingredient, for example carbon dioxide, that may or may not comprise at least one further component. The terms "a" or "an" do not exclude plurality, i.e. the singular forms "a", "an" and "the" should be understood as to include plural referents unless the context clearly indicates all requires otherwise. In other words, all references to singular characteristics all limitations of the present disclosure shall include the corresponding plural characteristics or limitation, and vice versa, unless explicitly specified otherwise or clearly implied to the contrary by the context in which the reference is made. The terms "a", "an" and "the" hence have the same meaning as "at least one" or as "one or more" unless defined otherwise. For example, reference to "an ingredient" includes mixtures of ingredients and the like.

The terms "essentially", "about", "approximately", "substantially" and the like in connection with an attribute or value as disclosed herein include the exact attribute or the precise value as well as any attribute or value typically considered to fall within a normal range of variability accepted in the technical field concerned. For example, the term "about" as used herein referring to a specific value or range should be understood that such value or range also includes deviations thereof, for example by up to 1 % or up to 2 % or up to 3 % or up to 4 % or up to 5 % (abs.).

The term "treatment" as used herein should be understood to include, unless otherwise noted, a therapeutic intervention to a subject capable of effecting/providing a cure or alleviation of a disease, condition, disorder or symptom in such subject. Furthermore, the term "treatment" as used herein should be understood to include, unless otherwise noted, i.e. an improvement, amelioration, control, control of progression or reduction of such disease, condition, disorder or symptom in a subject.

The term "prevention" as used herein, unless otherwise noted, should be understood to include a prophylactic intervention to a subject who does not exhibit signs or symptoms of a disease, disorder or condition, or does not exhibit the full scale of such signs, symptoms, disorder or condition yet, but who is expected or anticipated to likely exhibit such signs or symptoms in the absence of prevention. Furthermore, a prevention or preventive treatment may at least lessen or partly ameliorate such expected symptoms or signs.

The term "gaseous" as used herein, especially in connection with the compositions comprising carbon dioxide as disclosed herein, unless otherwise noted, should be understood to refer to a compound or composition that is present in the state of a gas and not in the state of a liquid or solid. The term "gaseous", however, should be understood to also include mixtures, specifically dispersions of a gaseous compound or composition with droplets of at least one liquid or particles of at least one solid substance.

The term "pressurized" as used herein, especially in connection with the compositions comprising carbon dioxide as disclosed herein, unless otherwise noted, should be understood to refer to a compound or composition, specifically a liquid or gaseous composition, that is present or provided under a pressure which is higher than the (surrounding) atmospheric pressure and/or higher than the standard atmospheric pressure of approximately 1.013 bar.

In a broad sense, the present invention relates to carbon dioxide (CO₂) or a composition comprising carbon dioxide (CO₂) for use in the treatment or prevention of a disorder or condition associated with headache, or a disorder or condition involving the trigeminal nervous system in a subject, or for use in in the treatment or prevention of allergic rhinitis, rhinosinusitis, trigeminal neuralgia, post-traumatic headache, temporomandibular joint disorder or epilepsy and seizures in a subject, wherein the composition comprising carbon dioxide is administered to the nasal cavity of the subject by intranasal administration in the form of a gaseous composition comprising carbon dioxide.

In a first aspect, the present invention relates to a pressurized composition comprising carbon dioxide (CO₂) for use
- in the treatment or prevention of a disorder or condition associated with headache, or a disorder or condition involving the trigeminal nervous system in a subject, or
- in the treatment or prevention of allergic rhinitis, rhinosinusitis, trigeminal neuralgia, posttraumatic headache, temporomandibular joint disorder, epilepsy or seizures in a subject,

wherein the composition comprising carbon dioxide is administered to the nasal cavity of the subject by intranasal administration in the form of a gaseous composition comprising carbon dioxide, and
wherein the gaseous composition comprising carbon dioxide is administered to the nasal cavity of the subject via both nostrils of the subject.

The pressurized compositions comprising carbon dioxide, more specifically the pressurized pharmaceutical compositions comprising carbon dioxide, for use according to the present invention are useful for the treatment or prevention, preferably for the treatment of a disorder or condition associated with headache, or a disorder or condition involving the trigeminal nervous system in a subject, or for the treatment or prevention, preferably for the treatment of allergic rhinitis, rhinosinusitis, trigeminal neuralgia, posttraumatic headache, temporomandibular joint disorder, epilepsy or seizures in a subject. In particular embodiments, the present compositions comprising carbon dioxide for use according to the present invention are useful for the treatment or prevention, preferably for the treatment of a disorder or condition associated with headache or a disorder or condition involving the trigeminal nervous system in a subject.

The term "disorder associated with headache" as used herein may comprise any disorder listed in the 3rd edition of the International Classification of Headache Disorders (ICHD-3) published by the Headache Classification Committee of the International Headache Society (IHS) (Cephalalgia 2018, Vol. 38(1) 1-211).The corresponding contents of the ICHD-3 are incorporated herein by reference.

In some embodiments of the compositions for use according to the present disclosure, the disorder or condition associated with headache is migraine. Migraine is a common, potentially disabling disorder characterized by episodic attacks of mild, moderate, or severe headache, with a variety of neurological and systemic manifestations including photophobia, phonophobia, cutaneous allodynia, nausea, cognitive impairment and fatigue. It is classified as occurring with or without aura, and as either episodic or chronic (headache for ≥15 days/month for 3 months, of which at least 8 days/month had features of migraine).

Accordingly, in some embodiments of the compositions for use according to the present disclosure, the disorder or condition to be treated or prevented is migraine with aura. In alternative embodiments, the disorder is migraine without aura. In some embodiments of the compositions for use according to the present disclosure, the disorder or condition may be episodic migraine. In some further embodiments, the disorder or condition may be chronic migraine.

In some embodiments, the disorder or condition associated with headache to be treated or prevented is migraine with brainstem aura (corresponding to the terms "basilar artery migraine" or "basilar migraine" originally used in the literature; however, since involvement of the basilar artery is unlikely, the term "migraine with brainstem aura" is preferred and used herein, accordingly).

In further embodiments, the disorder or condition associated with headache that may be treated or prevented by the compositions for use according to the invention is tension-type headache (TTH).

In some embodiments, the disorder or condition associated with headache to be treated or prevented may be a trigeminal autonomic cephalalgia (TAC).

In some embodiments, the disorder or condition to be treated or prevented associated with headache may be selected from the group consisting of painful lesions of the cranial nerves and other facial pain.

In some embodiments, the disorder or condition associated with headache to be treated or prevented may be headache attributed to psychiatric disorder.

In some embodiments, the disorder or condition to be treated or prevented by administration of the compositions comprising carbon dioxide for use according to the present invention affects or involves the trigeminal nervous system, specifically the trigeminal nerve.

In some embodiments, the disorder or condition that may be treated or prevented is a temporomandibular disorder, e.g. temporomandibular dysfunction.

In some embodiments, the disorder or condition that may be treated or prevented by administration of the compositions comprising carbon dioxide for use according to the present invention may be epilepsy and/or seizures, such as epileptic seizures or non-epileptic seizures.

In other embodiments, the disorder or condition associated with headache that may be treated or prevented is cluster headache.

In yet other embodiments, the disorder or condition associated with headache that may be treated or prevented is chronic orofacial pain.

In another embodiment, the disorder or condition associated with headache that may be treated or prevented is acute pain, or pain during an acute phase of migraine.

In some preferred embodiments, the disorder or condition that may be treated or prevented by administration of the compositions comprising carbon dioxide for use according to the present disclosure is allergic rhinitis.

In another embodiment the disorder or condition that may be treated or prevented is traumatic brain injury and/or concussion and/or associated symptoms.

In some embodiments, the disorder or condition that may be treated or prevented is rhinosinusitis. In some other embodiments, the disorder or condition that may be treated is trigeminal neuralgia.

In yet other embodiments, the disorder or condition that may be treated or prevented by administration of the compositions comprising carbon dioxide for use according to the present disclosure is post-traumatic headache.

In some embodiments, the disorder or condition that may be treated or prevented by administration of the compositions comprising carbon dioxide for use according to the present disclosure as referred to above, may occur in the form of a combination of a plurality of two or more of the above-described disorders or conditions.

The term "composition comprising carbon dioxide (CO₂)" for use as disclosed herein, in the broadest sense, refers to a composition which comprises carbon dioxide (CO₂; CAS-Nr. 124-38-9) and optionally at least one further constituent. In some embodiments, the compositions for use according to the present invention may comprise further gases, preferably further physiologically or pharmaceutically acceptable gases, for example, air, nitrogen, nitric oxide, nitrous oxide, methoxyfluorane, oxygen, and/or flurane, noble gases such as helium, neon, argon and/or xenon. In some embodiments, the present compositions comprising carbon dioxide, particularly the pressurized compositions comprising carbon dioxide may comprise carbon dioxide in a concentration of from about 1 mol-% to about 99.99 mol-%, or of at least about 50 mol-% or of at least about 75 mol-% or of at least about 80 mol-% or of at least about 90 mol-% to about 99,99 mol-%, or of from about 95 mol-% or from about 98 mol-% to about 99.99 mol-% or to about 99.95 mol-% or of from about 95 mol-% to about 99.9 mol-% (all with regard to the total composition), particularly when used in gaseous form as described in further detail below.

In alternative embodiments, however, the compositions comprising carbon dioxide for use according to the present invention may comprise carbon dioxide in an amount of even higher than 99 mol-% or of even higher than about 99.9 mol-% and, accordingly may consist or essentially consist of carbon dioxide comprising only trace amounts of up to about 1 mol-% or of up to about 0.1 mol-% or of about 0.01 mol-% of other constituents (impurities) such as nitric oxide or others.

The compositions comprising carbon dioxide as described above, especially when provided in the form of a composition consisting or essentially consisting of carbon dioxide, may initially be provided in the form of a liquid or, in other words, in compressed and/or condensed form. In some embodiments, the compositions comprising carbon dioxide may be initially provided at a pressure of up to about 60 bar or of up to about 58 bar, for example, selected within the range of from about 40 bar to about 60 bar, or from about 45 bar to about 58 bar, or from about 50 bar to about 57 bar. For the avoidance of doubt, however, it should be noted that the pressure ranges as defined above refer to the compositions comprising carbon dioxide for use according to the present disclosure as initially provided, for example in a reservoir as described in further detail below, and that these pressure ranges do not refer to the "gaseous composition comprising carbon dioxide" to be administered to the nasal cavity of a subject as will also be described in further detail below.

The term" gaseous composition comprising carbon dioxide" to be administered to the nasal cavity of a subject via both nostrils of the subject according to the present disclosure refers to a gaseous composition comprising carbon dioxide that may be provided by evaporation and/or decompression of the compositions comprising carbon dioxide as initially provided. In the context of the present disclosure, such evaporation and/or decompression of a composition comprising carbon dioxide from the pressurized or, in other words, compressed state and, in some embodiments, condensed and/or liquid state, to a less pressurized and, in some embodiments, less pressurized and or gaseous state by adiabatic expansion of the initial composition comprising carbon dioxide in a more pressurized state is also referred to as "preconditioning". Likewise, the gaseous composition comprising carbon dioxide for use according to the present disclosure in its fully or partly decompressed state may also be referred to as a or the composition comprising carbon dioxide for use according to the present disclosure in a preconditioned state. Accordingly, in some embodiments the gaseous composition for use according to the present invention is administered in preconditioned form.

Such gaseous compositions comprising carbon dioxide may be provided or administered to the subject at a pressure of up to about 20 bar or of up to about 15 bar, for example, selected within the range of from about 1.5 bar or of from about 2 bar to about 10 bar or to about 8 bar, or from about 2 bar or from about 4 bar to about 6 bar (as measured at the exit of the applicator device as described in detail further below according to the second aspect of the invention).

In some embodiments, the gaseous compositions comprising carbon dioxide for use according to the present invention may be administered to the nasal cavity of the subject at a temperature being in the range of from about -10 °C to about 22 °C (or ambient temperature) or of from about -4 °C to about 22°C or of from about 0 °C or from about 5 °C to about 18 °C or of from 5 °C to about 15 °C (as measured at the exit of the applicator device during the first second of initiating the application as described in detail further below in connection with the second aspect of the invention).

In some embodiments, the pressurized gaseous compositions comprising carbon dioxide for use according to the present invention may be administered to the nasal cavity of the subject in the form of doses, more specifically in the form of discrete doses and preferably not in the form of a continuous stream of the gaseous compositions. Accordingly in particular embodiments, the gaseous composition according to the present invention is administered in the form of at least one discrete dose, for example in the form of one or two or three or four consecutive discrete, discontinuous doses. In preferred embodiments, however, as described in further detail below, the gaseous composition according to the present invention is administered in the form of one discrete dose.

In some embodiments, the pressurized gaseous compositions comprising carbon dioxide for use according to the present invention may be administered to the nasal cavity of the subject in the form of one or more metered doses. The term "metered" as used herein, unless otherwise stated, refers to a specific and, in some embodiments, reproduceable and/or adjustable amount or volume of the gaseous composition. In some embodiments, the gaseous composition comprising carbon dioxide may be administered to the nasal cavity of the subject in the form of a metered and/or discrete dose having a volume selected within the range of from about 1 mL (cm³) to about 1200 mL or from about 5 mL to about 600 mL or from about 10 mL to about 500 mL, or of from about 10 mLto about 400 mL or of from about 20 mL to about 300 mL as described in further detail below.

In some embodiments, even when being administered in the form of a (discrete) dose, the gaseous composition comprising carbon dioxide may be administered to the nasal cavity of the subject at a flow rate of from about 1 mL/s (mL per second) to about 30 mL/s, or of from about 3 mL/s to about 30 mL/s or from about 5 mL/s or of from about 10 mL/s to about 25 mL/s, or from about 10 mL/s to about 20 mL/s or of from about 12 mL/s to about 18 mL/s.

In some embodiments, a discrete and/or metered dose of the gaseous composition comprising carbon dioxide may comprise carbon dioxide in an amount of from about 20 mg to about 700 mg, or of from about 45 mg to about 530 mg or of from about 70 mg or of from about 85 mg to about 400 mg, or of from about 85 mg to about 350 mg, whereas the remaining gaseous constituents may be air or other physiologically and/or pharmaceutically acceptable gases as described above. In some embodiments, the gaseous compositions, especially when administered in the form of doses, particularly in the form of discrete doses, such as in the form of one discrete dose, as described in further detail below, may be administered to the nasal cavity of the subject within a period of from about 1 s to about 30 s, or of from about 3 s or from about 4 s to about 25 s or to about 20 s, or of from about 5 s to about 15 s or from about 5 s to about 12 s (as measured from the beginning to the end of the administration of the gaseous composition into the nasal cavity of the subject).

In some embodiments, the pressurized compositions comprising carbon dioxide for use according to this first aspect of the present disclosure also comprise compositions comprising carbon dioxide (CO₂) for use
- in the treatment or prevention of a disorder or condition associated with headache, or a disorder or condition involving the trigeminal nervous system in a subject, or
- in the treatment or prevention of allergic rhinitis, rhinosinusitis, trigeminal neuralgia, post-traumatic headache, temporomandibular joint disorder, epilepsy or seizures in a subject,

wherein the composition comprising carbon dioxide is initially provided in pressurized and/or condensed form and is administered to the nasal cavity of the subject by intranasal administration in the form of a less pressurized and/or gaseous composition comprising carbon dioxide, and
wherein the gaseous composition comprising carbon dioxide is administered to the nasal cavity of the subject via both nostrils of the subject.

For the avoidance of doubt, it should be understood that the present compositions comprising carbon dioxide, particularly the present compositions comprising carbon dioxide for use according to the present invention, after introduction or administration to the nasal cavity of a subject are at atmospheric pressure or, if at all, only slightly elevated pressure as present in the nasal cavity of such subject.

The term "subject" as used herein is to be understood as to preferably relating to a warm-blooded animal or human subject, preferably to a human subject. In some embodiments, the subject as referred to herein may be an adult human subject, i.e. a human of at least 18 years of age, or a non-adult or infant subject of less than 18 or even less than 12 or 6 years of age.

In specific embodiments, the term "subject" as used herein refers to a subject being poor- to non- responsive to treatment with one or more pain related treatment options, e.g. triptans and NSAIDs/paracetamol. In further specific embodiments, such subject is non-responsive or poorly responsive to treatment with at least one triptan, specifically at least one triptan selected from the group of triptans comprising sumatriptan, almotriptan, eletriptan, frovatriptan, naratriptan, rizatriptan, and zolmitriptan.

In some embodiments, the subject to be administered with the compositions for use according to the present disclosure may exhibit elevated salivary levels of at least one prostaglandin, compared to a healthy control subject. The at least one prostaglandin may be selected from the group consisting of PGD2, PGF2, TXA2 and combinations thereof.

In some embodiments, the subject to be administered with the compositions for use according to the present disclosure may exhibit a salivary level of at least one prostaglandin after treatment which is lower than the salivary level of said at least one prostaglandin before treatment. The at least one prostaglandin may be selected from the group consisting of PGD2, PGF2, TXA2 and combinations thereof.

In some embodiments, the subject to be administered with the compositions for use according to the present disclosure may exhibit elevated salivary levels of CGRP, of vasoactive intestinal peptide (VIP), or both, compared to a healthy control subject.

In some embodiments, the subject to be administered with the compositions for use according to the present disclosure may exhibit a salivary level of CGRP, VIP, or both after treatment, which is lower than the salivary level of CGRP, VIP or both before treatment.

The term "elevated levels" refers to an elevation of at least 5%, preferably at least 10%, or at least 20%, relative to the baseline value. The term "lower level", as used herein, refers to a reduction of at least 5%, or at least 10% or at least 20%.

The pressurized composition comprising carbon dioxide or, more specifically, the pressurized gaseous composition for use according to the present disclosure is administered to the nasal cavity of the subject by intranasal administration or application. The term "nasal cavity" of a subject as used herein means, unless otherwise stated, the air-filled space in the head of a subject above and behind the nose in the middle of the head of such subject. The nasal cavity comprises two cavities each being a continuation of each nostril and being divided by nasal septum. The lateral walls of the nasal cavity or, more specifically, of each of the two cavities mainly consists of the maxilla. The roof of each nasal cavity is formed by the nasal bone, the nasal septum and the maxilla in some parts. The nasal cavities also include the respective sinuses, namely the frontal sinuses, sphenoidal sinuses, ethmoidal sinuses and maxillary sinuses. The floor of the nasal cavities is formed by the bones of the hard palate. The term "nasal cavity" as used herein preferably comprises the respiratory and the olfactory segment. The nasal cavity or, more specifically both nasal cavities together in many cases have a volume in the range of from about 28 cm³ to about 38 cm³ in healthy adult humans and of from about 2 cm³ to about 5 cm³ in healthy infants. Furthermore, the nasopharyngeal cavity of a healthy adult human typically ranges of from about 28 cm³ to about 45 cm³ and of from about 3 cm³ to about 9 cm³ for a healthy infant.

In some embodiments, the pressurized compositions for use according to the present disclosure, more specifically in the form of a gaseous composition, may be administered to the nasal cavity of the subject as described above and, in addition, to the nasopharynx and/or the pharynx of such subject. Accordingly, in some embodiments, the gaseous composition, may be administered to the nasal cavity of the subject and at least partially the nasopharynx and/or to the pharynx of such subject such that the gaseous compositions may be introduced to the nasal cavity and also to a partial inner volume or to the entire inner volume of the nasopharynx and/or pharynx of the subject. In some embodiments, however, the gaseous composition may not be administered to or contacted with elements of the airway of the subject located beyond the pharynx of such subject, for example the larynx of such subject.

The term "intranasal" as used herein, for example, in connection with the terms administration or application or injection or dosage and the like refers to a route of administration, specifically of the gaseous compositions comprising carbon dioxide for use according to the present invention in which such gaseous composition is introduced into the nasal cavity of a subject, or, more specifically into both nasal cavities via both nostrils (i.e. through the left and the right nostril, the terms "via" and "through" being used synonymously herein) of the subject. In further specific embodiments, the gaseous composition comprising carbon dioxide for use according to the present disclosure is administered to the nasal cavity or, more specifically, to both nasal cavities of the subject simultaneously via both nostrils of the subject.

The term "simultaneous" as used herein in connection with the above-mentioned intranasal administration, unless otherwise stated, means that the gaseous composition is introduced to both nostrils of the subject at least partly at the same time. This means, that, for example, administration into one nostril may be initiated at a specific time while administration to the other nostril is initiated at a specific later time, however, before termination of the administration into or via the first nostril. In some preferred embodiments, the term "simultaneous" means that administration or introduction of the present gaseous composition to both nostrils is at least initiated at the same time and, in further preferred embodiments, is also terminated at the same time.

In some embodiments, such intranasal administration of the gaseous composition comprising carbon dioxide for use according to the present disclosure to the nasal cavity or cavities of a subject may be implemented in a way that during administration of the gaseous composition comprising carbon dioxide the subject continues to inhale or, in other words, continues to breathe in and optionally to breathe out, specifically through the mouth of the subject.

In some embodiments, however, such intranasal administration of the gaseous composition comprising carbon dioxide for use according to the present disclosure to the nasal cavity or cavities of a subject may be implemented in a way that during administration of the gaseous composition comprising carbon dioxide the subject discontinues to inhale or to breathe in or out and, accordingly, does not inhale or breathe in or out or, in other words, refrains from inhaling or breathing in or out, at least for part of the time of the administration. In some particular embodiments, during administration of the present gaseous composition comprising carbon dioxide the subject does not inhale (breathe)/refrains from inhaling/breathing through the nose or through the nose and mouth.

In some embodiments, the gaseous composition comprising carbon dioxide for use according to the present invention is administered in the form of a first dose, specifically in the form of a first metered dose administered within a treatment period, particularly within an initial treatment period of about 10 minutes or of about 15 minutes or of about 20 minutes, specifically administered within an (initial) treatment period of 10 minutes (min) or of about 15 minutes.

In some embodiments, the gaseous composition comprising carbon dioxide for use according to the present invention is administered in the form of a sole dose or, in other words, in the form of a single dose, particularly in the form of a single metered dose, administered within the treatment period of about 10 min or of about 15 min or of about 20 min, respectively.

The term "treatment period" or "initial treatment period" as used herein, unless otherwise stated, refers to a duration of time starting from the initiation of the administration of a first dose of the composition comprising carbon dioxide for use according to the present invention to the nasal cavity or cavities of the subject by intranasal application as described above and ending about 10 min, for example, from about 9 min to about 11 min, more specifically, ending 10 minutes after such initiation of administration.

In some embodiments, the gaseous composition comprising carbon dioxide for use according to the present invention is administered in the form of a sole dose or, in other words, in the form of a single dose administered within the treatment period of about 11 min or of about 12 min or of about 13 min or of about 14 min or of about 15 min or of about 20 min, respectively.

In some embodiments, the gaseous compositions comprising carbon dioxide for use according to the present invention may be administered to the subject in a way that a second dose of the composition comprising carbon dioxide is administered at least about 20 min or at least about 15 min or at least about 14 min or at least about 13 min or at least about 12 min or at least about 11 min or at least about 10 min after administration of the first dose, especially in cases in which the disorder or condition associated with headache as experienced by the subject is still present or partly present about 20 min or about 15 min or about 14 min or about 13 min or about 12 min or about 11 min or about 10 min, respectively, after administration of the first dose.

In specific embodiments, the compositions comprising carbon dioxide for use according to the present disclosure may be administered in the form of a second dose at least 10 min after administration of the first dose, especially in cases in which the disorder or condition associated with headache as experienced by the subject is still present or at least partly present 10 min after administration of the first dose.

Accordingly, in some embodiments the compositions comprising carbon dioxide for use according to the present disclosure may be administered in a way comprising administration of a second dose not earlier than 10 minutes after administration of the first dose. In many cases, however, the symptoms will improve after application of the first dose. If, however, symptoms of the disorder or condition associated with headache or, in other words, the attack persists, a second dose may be administered. Preferably, the second dose may be administered not earlier than 11 minutes after the first dose. In other embodiments, the second dose may be administered not earlier than 11 minutes, or not earlier than 12 minutes, or not earlier than 13 minutes, or not earlier than 14 minutes, or not earlier than 15 minutes, or not earlier than 20 min after the first dose.

In contrast to the term "treatment period" as used herein and as defined above, the term "administration period" refers to a duration of time during which the gaseous compositions comprising carbon dioxide for use according to the present invention are actually administered to the nasal cavity (or the nasal cavities) of the subject starting from the beginning of the actual intranasal administration or application of the (first and preferably sole) dose of the composition and ending with the termination of such actual application or administration. Furthermore, the term "exposure period" refers to a duration of time during which the gaseous compositions comprising carbon dioxide are actually contacting at least part of the mucous membranes of the nasal cavity (or nasal mucosa as used herein synonymously) of the subject starting from the initial contact of the administered composition comprising carbon dioxide and ending with the termination of such contact, while at the same time exerting it's physical and/or physiological effects to the nasal cavity, related tissues and nerve endings, such as applying a gentle cooling and others.

In this context it should be noted that the administration of the compositions comprising carbon dioxide for use according to the present invention into the nasal cavity of a subject may lead to the cooling of the mucous membrane of the nasal cavity of the subject, particularly of the affected nerve tissue such as the trigeminal nerve or, more specifically, the corresponding Aδ- and C-fiber neurons. It should be furthermore noted that the administration of the compositions comprising carbon dioxide for use according to the present invention into the nasal cavity of a subject may cause a modulation or change of the intracellular pH-value, more specifically, a decrease of the intracellular pH-value of the involved nerve cells as well as a subsequent modulation of the involved nerve cells including but not limited to trigeminal nerve cells, for example the corresponding Aδ- and C-fiber neurons.

As, in preferred embodiments, the administration or application of the compositions comprising carbon dioxide for use according to the present disclosure is conducted in the form of the administration of a dose or bolus of such composition, and not in the form of a continuous administration in which the gaseous composition comprising carbon dioxide is administered in the form of a continuous stream of such gaseous composition, the administration period is much shorter than the treatment and exposure period and may range from about 1 s to about 30 s, or from about 3 s or from about 4 s to about 25 s or to about 20 s, or from about 5 s to about 15 s, or from about 5 s to about 12 s as already described above.

Furthermore, in some embodiments of the compositions comprising carbon dioxide for use according to the present disclosure the intranasal administration comprises exposing (contacting) at least a part of the nasal mucous membranes of the subject with the gaseous composition comprising carbon dioxide for a duration (exposure period) of from about 1 s to about 90 s or even 120 s, or from about 1 s to about 60 s.

In some further embodiments, the duration of the exposure period as described above exceeds the duration of the corresponding administration period, in many cases for up to about 110 s or for example up to about 80 s, or for up to 50 s.

The term "dose" as used herein, specifically with regard to the compositions comprising carbon dioxide, more specifically the gaseous compositions comprising carbon dioxide for use according to the present disclosure may refer to the amount of such composition, preferably gaseous composition that is actually administered into the nasal cavity or cavities of the subject during one administration period and that is sufficient to generate a concentration of carbon dioxide within the nasal cavity of the subject that ranges from about 1 mol-% to about 99.9 mol-%, or of at least about 50 mol-% or of at least about 75 mol-% to about 99,9 mol-%, or of from about 80 mol-% to about 99.9 mol-%. As already mentioned above and in view of the volume of the nasal cavity of the subject such dose may have a volume selected within the range of from about 1 mL (1 cm³) to about 1200 mL, or of from about 5 mL to about 600 mL or of from about 10 mL to about 500 mL or to about 400 mL, or of from about 20 mL to about 300 mL, or as already described above.

In some embodiments of this first aspect of the invention, the compositions comprising carbon dioxide for use according to the present invention, more specifically the compositions comprising carbon dioxide in the form of a gaseous composition, may be administered using an applicator device. The term "applicator device" as used herein, unless otherwise stated, may be understood to comprise a device which is suitable for or adapted or arranged for or the application or administration of the compositions comprising carbon dioxide for use according to the present invention wherein the composition comprising carbon dioxide is administered to the nasal cavity of the subject by intranasal administration in the form of a gaseous composition comprising carbon dioxide, and wherein the gaseous composition comprising carbon dioxide is administered to the nasal cavity of the subject via both nostrils of the subject.

In some embodiments, the such applicator device may be a hand-held device that may be held and/or operated with one or two hands of a user, specifically the subject or in other words, that may be dimensioned with regard to, for example, its outer dimensions and its weight, that it may be conveniently held and operated by a user, specifically by the subject, also in cases in which the subject may be impaired by a disorder or condition, for example as described in detail above. In exemplary embodiments, the applicator device in its fully assembled and ready for use state may have an overall length (as measured along main axis connecting its proximal end with its distal end) of from about 5 cm to about 25 cm or of from about 7.5 cm to about 20 cm. In further exemplary embodiments, the applicator device in its fully assembled and ready for use state may have an overall maximum diameter (as measured perpendicular to its main axis) of from about 1 cm to about 20 cm or of from about 2 cm to about 10 cm or to about 7.5 cm. In further exemplary embodiments, the applicator device in its fully assembled and ready for use state may have an overall weight of up to about 500 g (gram) or within the range of from about 5 g to about 250 g or of from about 10 g to about 150 g.

In some embodiments, the applicator device is adapted or arranged for the generation of an atmosphere within the nasal cavity or cavities of the subject or, as discussed above, within the nasal cavity and at least partially within the nasopharynx and/or pharynx of the subject, comprising carbon dioxide in a concentration selected within the range of 1 mol-% to about 99.99 mol-%, or of at least about 50 mol-%, or of at least about 70 mol-%, or of at least about 75 mol-% or of at least about 80 mol-% or of at least about 90 mol-% to about 99 mol-% or to about 99.9 mol-% or to about 99,99 mol-%, or of from about 95 mol-% or from about 98 mol-% to about 99.99 mol-% or to about 99.95 mol-% or of from about 95 mol-% to about 99.9 mol-% (all with regard to the total composition). In particular embodiments, the applicator device is adapted or arranged for the generation of an atmosphere in the nasal cavity and optionally the nasopharynx and/or the pharynx of the subject which is saturated with carbon dioxide. In particular embodiments, such concentration may be present for a period of time selected in the range of from about 1 s to about 90 s or even 120 s, or from about 1 s to about 60 s.

In particular embodiments, the pressurized compositions comprising carbon dioxide for use according to the present invention, specifically the pressurized gaseous compositions comprising carbon dioxide may be intranasally introduced to applied or administered to the subject via both nostrils of the subject using an applicator device, wherein the applicator device comprises:
a) a storage unit for holding and/or providing the composition comprising carbon dioxide in pressurized form;
b) a pre-conditioning unit comprising a pre-conditioning chamber fluidically connected with the reservoir for transforming the carbon dioxide in pressurized form into the gaseous composition comprising carbon dioxide; and
c) an application unit fluidically connected with the pre-conditioning unit, specifically with the pre-conditioning chamber, for introducing the gaseous composition comprising carbon dioxide into the nasal cavity of the subject via both nostrils of the subject; and optionally
d) a housing for holding the storage unit, the conditioning unit, the application unit and optionally further functional units of the applicator device.

The applicator device suitable for or adapted or arranged for the administration of the compositions comprising carbon dioxide for use according to the present disclosure, as a first functional unit, may comprise a storage unit which is suitable for holding and/or providing the composition comprising carbon dioxide in pressurized and or condensed form as already described above. Such storage unit, in some embodiments, may comprise a reservoir or other container or hollow body suitable for holding the composition comprising carbon dioxide in pressured form as described in further detail above, for example at a pressure of up to about 60 bar or of up to about 58 bar, for example, selected within the range of from about 40 bar to about 60 bar, or from about 45 bar to about 58 bar, or from about 50 bar to about 57 bar. In particular embodiments, such reservoir comprises the composition comprising carbon dioxide in condensed form (in the form of a liquid).

Such reservoir, in some embodiments may comprise the carbon dioxide to be administered to the subject in (essentially) pure form or in the form of a mixture comprising carbon dioxide in an amount of from about 1 to about 99.9 mol-% or in an amount of at least about 50 mol-% or of at least about 75 mol-% with regard to the total amount of such mixture as described in detail above. In some embodiments, such reservoir or container, in view of the pressure of the compositions comprising carbon dioxide contained or to be provided therein, may be provided in a form suitable for the safe and reliable storage and dispensing of such pressurized compositions. In some embodiments, the reservoir or container may comprise or substantially consist of a metal such as steel, stainless steel, aluminum, brass and alloys thereof or may comprise or substantially consist of a dimensionally stable polymeric material, such as a thermoplastic material, for example selected from ethylene propylene diene monomer rubber (EPDM), Tecasint, Tecaflon, acrylonitrile butadiene styrene (ABS), a polyethylene, e.g. PE 10, a polyamide, e.g. PA 6, polypropylene homopolymer, e.g. PPH medical or polytetrafluorethylene (PTFE).

In some embodiments, the reservoir or container as comprised by the storage unit may have an inner volume of from about 1 mLto about 500 mL, or of from about 1 mLto about 250 mL or to about 150 mL, or of from about 1.5 mL to about 25 mL or of from about 2 mLto about 15 mL or to about 10 mL. In some embodiments, such reservoir or container may be provided in the form of a replaceable reservoir or container. In these embodiments the application device comprises a replaceable reservoir or container. The term "replaceable" as used herein in connection with the reservoir or container of an applicator device shall be understood, unless otherwise stated, as a reservoir that at a certain point of time, for example when emptied, may be disconnected or removed from the applicator device and may be replaced by another reservoir or container holding the composition comprising carbon dioxide. In these embodiments, the storage unit may comprise a connecting unit or connecting port for connecting to and establishing a fluidic connection between the (inner volume) of the reservoir or container and the one or more further functional units of the applicator device, for example the pre-conditioning unit.

In alternative embodiments, the application device may comprise a non-replaceable reservoir or container. In these embodiments, the reservoir or container holding the pressurized, preferably the condensed composition comprising carbon dioxide may be firmly attached or, in other words, inseparably attached to the further functional units of the applicator device such that the reservoir or container may not be removed or disconnected from the applicator device in a non-destructive manner. In these embodiments, the applicator device may also be referred to as a disposable applicator device that may be disposed of together with the other functional units of the applicator device after use or at any given time, for example when the reservoir or container is partly or fully emptied.

The applicator device for the administration of the compositions for use according to the present invention, specifically for the intranasal administration of such compositions in gaseous form via both nostrils of a subject as a further functional unit comprises a pre-conditioning unit. Such pre-conditioning unit may be fluidically connected to the storage unit, more specifically to the reservoir or container as described above. The term "fluidically connected" or" in fluid communication" as used herein, unless otherwise stated, shall be understood to refer to a connection between a unit, or structural element to another unit or structural element which allows for the transfer of a material, specifically of a fluidic material such as a gas and/or a liquid, from one unit or structural element to at least one other unit or structural element, preferably substantially without loss of such transferred material, by establishing a fluidic connection. Such fluidic connection, in exemplary embodiments, may comprise a tubing or pipe or other sealed channel and may comprise further fluidic elements such as valves, connectors, pressure reducers, membranes, tubing, channels and the like.

The pre-conditioning unit as described above, in some embodiments, is suitable or adapted or arranged for transforming the pressurized composition comprising carbon dioxide in pressurized form into a gaseous composition comprising carbon dioxide (in less pressurized form). In other words, the pre-conditioning unit as comprised by the applicator device is suitable for transforming the composition comprising carbon dioxide as initially provided in compressed and/or condensed form by the storage unit into the gaseous composition comprising carbon dioxide corresponding to the form in which the compositions comprising carbon dioxide for use according to the present invention are to be administered intranasally to the subject via both nostrils.

In some embodiments, the pre-conditioning unit is suitable or adapted or arranged for transforming the composition comprising carbon dioxide in pressurized and/or condensed form into a gaseous composition comprising carbon dioxide in less pressurized and/or gaseous form or as used herein in an alternative description, in pre-conditioned form. In this less pressurized, gaseous and pre-conditioned form the compositions for use according to the present invention are especially useful for the intranasal application into the nasal cavity of a subject, especially when administered via both nostrils of the subject.

In some embodiments, the pre-conditioning unit comprises a preconditioning chamber. In some embodiments, such pre-conditioning chamber may comprise a hollow body into which the compositions comprising carbon dioxide in compressed and/or condensed form may be transferred and which allows for the expansion, more specifically, the adiabatic expansion of such compressed compositions into a less compressed and gaseous state (in which the gaseous compositions for use according to the present invention may be administered intranasally to the subject).

In some embodiments, the hollow body of such pre-conditioning chamber is useful for generating and temporarily accommodating the gaseous compositions comprising carbon dioxide to be administered to the subject. In some embodiments, the preconditioning chamber may have an inner volume of from about 1 mL to about 10 mL or of from about 2 mL to about 6 mL. In further embodiments, the pre-conditioning unit, specifically the pre-conditioning chamber has an inlet fluidically connected to the storage unit, more specifically to the reservoir or container of the storage unit, and an outlet fluidically connected to the application unit.

In some embodiments, the inlet of the pre-conditioning chamber may be fluidically connected to the storage unit, more specifically to the reservoir or container of the storage unit via a pressure reducer or a valve such as a metering valve. In the operational mode of the applicator device, upon establishing the fluidic connection between the storage unit, more specifically the reservoir or container of the storage unit, and the precondition unit, more specifically the pre-conditioning chamber of the pre-conditioning unit, upon actuation the pressurized and/or condensed composition is allowed to (adiabatically) expand and thereby to decompress into the pre-conditioning chamber. In some embodiments, this may be accompanied by contact between the composition comprising carbon dioxide and the air initially present in the pre-conditioning chamber. In some embodiments, this may be accompanied by a drop of pressure as well as a drop of temperature of the resulting gaseous compositions comprising carbon dioxide. In further embodiments, this may be accompanied by a (partial) reaction between the carbon dioxide provided in the composition for use with the air, more specifically the humidity of the air initially present in the pre-conditioning chamber resulting in the pre-conditioned compositions comprising carbon dioxide for use according to the present invention.

In some embodiments and without wishing to be bound by theory, the temperature of pre-conditioned gaseous composition (after expansion into the pre-conditioning chamber) will be lower than that of the initial pressurized composition due to the Joule-Thomson effect. This lowered temperature, however, in many cases will assimilate to the environmental temperature, according to the specific design of the pre-conditioning chamber. In some embodiments, the pre-conditioned gaseous composition (after expansion into the pre-conditioning chamber) may have a pressure within the range of from about 1.1 or 1.5 bar to about 20 bar, or from about 1.5 bar to about 10 bar or to about 10 bar or to about 8 bar or from about 4 to about 6 bar.

In some embodiments, the pre-conditioning unit, specifically the pre-conditioning chamber may be fluidically connected to the reservoir via a valve, specifically a metering valve. Such a metering valve may be helpful to provide discrete, adjustable and reproduceable doses of the gaseous compositions comprising carbon dioxide.

In further embodiments, however, the respective dose of the gaseous composition comprising carbon dioxide may be metered by the volume of the pre-conditioning chamber into which the initial (more compressed) composition may expand. In these embodiments, it may be useful to provide a valve between the (reservoir of the) storage unit and the (preconditioning chamber of the) pre-conditioning unit (herein sometimes also referred to as "reservoir expansion valve") that closes upon reaching a specific desired pressure of the gaseous composition within the pre-conditioning chamber. In these embodiments, it may be advantageous to provide for a separate dosage regulator tube or channel fluidically connecting the innervolume of the pre-conditioning chamber with the valve between the (reservoir of the) storage unit and the (pre-conditioning chamber of the) pre-conditioning unit.

As a further functional unit, the applicator device suitable and useful for the intranasal application of the compositions comprising carbon dioxide for use according to the present invention may comprise an application unit. Such application unit, in some embodiments, may be fluidically connected with the pre-conditioning unit, more specifically with the pre-conditioning chamber of the pre-conditioning unit and even more specifically with the outlet of the pre-conditioning chamber. In some embodiments, the application unit is suitable or adapted or arranged for introducing or applying or administering the gaseous composition comprising carbon dioxide into the nasal cavity of the subject via both nostrils of the subject.

In some embodiments, the application unit may comprise at least one nosepiece adapted or arranged for being introduced into both nostrils of the subject, more specifically, adapted or arranged for simultaneous introduction into both nostrils of the subject. In some embodiments, the application unit, more specifically the nosepiece of the application unit may be adapted for introduction into both nostrils of the subject, preferably for simultaneous insertion into both nostrils of the subject. In particular embodiments, the application unit, more specifically the nosepiece may comprise two oblong extensions, each of which shaped and dimensioned to be introduced into one of the two nostrils of the subject. In further preferred embodiments, the nosepiece or, more specifically, each of its two oblong extensions, comprises an application opening, preferably located at the most proximal end of the two extensions of the nosepiece, adapted to be introduced, specifically adapted to be simultaneously introduced into both nostrils of the subject.

In particular embodiments, the application unit is adapted or, in other words, may be shaped and dimensioned for sealing, preferably for tightly sealing both nostrils of the subject (when inserted into both nostrils of the subject). This may be achieved by a variety of potential technical solutions. In a first exemplary embodiment, the application unit, more specifically the oblong extensions may have a generally round or oval cross-sectional diameter and a conical overall shape with the smallest diameter near the corresponding exit opening. In other exemplary embodiments, the oblong extensions may have a generally cylindrical shape with at least one rounded and broadened section (i.e. at least one section with a larger gross-sectional diameter) adapted to a typical cross-sectional diameter of a human nostril.

In further embodiments, the application unit may comprise a replaceable and exchangeable plug to be mounted on top of the (oblong extensions) of the nosepiece of the application unit. In some embodiments, such plugs may be provided in the form of two independent plugs or, alternatively, in the form of one plug to be mounted on both oblong extensions simultaneously. Particularly in some of these embodiments, depending on the shape and dimensions of the particular plug or plugs to be mounted on the nosepiece, this may be advantageous, especially with regard to the adaption of the applicator device and nosepiece to the individual form and size of the nostrils of a particular subject and for realizing a tightly sealing fit of the nosepiece when introduced into the nostrils of a subject. In addition to that, such interchangeable plugs may be advantageous with regard to considerations in connection with hygiene.

In some embodiments, such plug or plugs may be made of a dimensionally stable material, such as a thermoplastic polymeric material. In alternative embodiments, however, such plug or plugs may be provided in flexible, specifically compressible form, for example in the form of a compressible or foamed polymeric material, for example ethylene propylene diene monomer rubber (EPDM), silicone and the like.

Accordingly, in some embodiments, the application unit comprises one or two plugs, preferably one or more flexible plugs, surrounding the application opening adapted for introduction and sealing closure of the nostrils of the subject.

In some embodiments, the application unit, more specifically the nosepiece of the application unit is designed, shaped and dimension to only contact the nostrils, more specifically the inner surface of the nostrils, of the subject to which the compositions comprising carbon dioxide for use according to the present invention is to be administered.

As an optional functional unit, the applicator device suitable and useful for the intranasal application of the compositions comprising carbon dioxide for use according to the present invention may further comprise a housing for holding the storage unit, the preconditioning unit, the application unit or at least a part of these functional units and optionally further functional units of the applicator device. In some preferred embodiments, the applicator device comprises such a housing which may be, in further embodiments, dimensioned and shaped to hold or encase at least the storage unit including an exchangeable or non-exchangeable reservoir or container, the preconditioning unit comprising a pre-conditioning chamber and at least a part of the application unit to provide for defined spatial relationship of these functional components relative to each other. In further embodiments, the optional housing provides for a surface advantageous for the handling of the preferably hand-held applicator device. Furthermore, such optional housing allows for the implementation and of further functional unit in the present applicator device.

In some embodiments, the application device may comprise as a further functional unit:
e) a control unit for controlling at least one of the amount, pressure, temperature and flow rate at which the gaseous composition comprising carbon dioxide is administered to the nasal cavity of the subject.

In some embodiments, such control unit may comprise valves, such as at least one metering valve for metering doses of the composition comprising carbon dioxide or the gaseous composition comprising carbon dioxide, respectively, as described above, or, for example, a reservoir expansion valve as described above and/or a reservoir expansion channel as described above. Such valves or metering valves, membranes or other functional elements, in some embodiments, may be fluidically connected with or comprised in the fluidic connection between, for example, the storage unit and the preconditioning unit or between the pre-conditioning unit and the application unit.

In some embodiments, the control unit may comprise a safety and release unit for the (controlled) release of the gaseous composition comprising carbon dioxide from the (pre-conditioning chamber of the) pre-conditioning unit into the application unit. Such safety and release unit, in some embodiments may comprise a further valve (for releasing the gaseous composition comprising carbon dioxide from the pre-conditioning chamber to the application unit). In some embodiments, such safety and release unit may further comprise one or more membrane or membranes suitable for the uni-directional release of the gaseous composition comprising carbon dioxide in the downstream direction towards the application unit. In some embodiments, such membrane or membranes may also be adapted for the selective passage of one or more specific gases as comprised by the gaseous composition, for example carbon dioxide. Such membranes are commercially available and may be purchased e.g. in the form of PTFE-based membranes such as e.g. GoreTex^{®}, SEPURAN^{®}. In some embodiments, the outlet of the pre-conditioning unit or chamber may be fluidically connected to the application unit via a valve, specifically via a metering valve and/or via a membrane.

As a further optional functional unit, the application device may further comprise:
f) an actuation unit for the actuation of the administration device such that the introduction/administration or release of the gaseous composition comprising carbon dioxide into the nasal cavity of the subject via both nostrils of the subject is initiated.

In some embodiments, such actuation unit may be adapted for manual actuation, preferably for manual actuation by the subject or other user. In further embodiments, actuation unit may comprise a button such as push button, or other form of actuator which is accessible from the outside of the applicator device, preferably from the outside of the optional housing of the applicator device for manual operation. In some further embodiments, such actuation unit or push button or other form of actuator may be coupled with the control unit, for example with a valve or metering valve for the generation of a dose of the gaseous composition comprising carbon dioxide to be administered to the subject upon actuation. In further embodiments, the actuation unit may comprise a trigger safety system by which the push button of the actuation unit may be secured.

The compositions comprising carbon dioxide for use according to the present invention, wherein the composition is administered to a subject in the form of a gaseous composition and wherein the composition is administered to the nasal cavity through both nostrils of the subject, especially when provided in connection with the applicator device as described in detail above, offer considerable advantages including, but not limited to
- the rapid and even application and distribution of the gaseous composition comprising carbon dioxide within the nasal cavity of the subject and, accordingly, the rapid and evenly distributed contact of carbon dioxide with the mucosa of the nasal cavity;
- the possibility to apply the gaseous composition comprising carbon dioxide at a reduced temperature which may be adjusted and controlled in a defined range resulting in a simultaneous cooling of the nasal mucosa for a defined amount of time;
- the possibility to apply the gaseous composition comprising carbon dioxide at a defined flow rate which reduces undesired effects, while allowing a rapid filling of the nasal cavity with CO₂;
- the reduction of outflow of the gaseous composition comprising carbon dioxide from the nasal cavity through one or both nostrils;
- the reduction of an excess of the gaseous composition comprising carbon dioxide reaching the airways beyond the nasal cavity, especially the lungs of the subject;
- the largely dispensability to rely on backflow of the gaseous composition comprising carbon dioxide through the pharynx;
- the reduction of adverse sensations and irritation experienced by the subject upon introduction of the gaseous composition comprising carbon dioxide into the nasal cavity;
- the avoidance of the risk of overdosing with the gaseous composition comprising carbon dioxide; and
- the administration of a gaseous composition comprising carbon dioxide to the nasal cavity of a subject in a safe, reliable, adjustable and reproducible manner.

Without wishing to be bound by theory, it is assumed that part of the pain relief as provided by the compositions comprising carbon dioxide for use according to the present invention may be due to a "thermodynamic effect", by cooling the intranasal tissue and the nerves embedded therein. Accordingly, in some embodiments, the present invention therefore relates to the pressurized compositions comprising carbon dioxide as described in detail above for use in the treatment or prevention of a disorder associated with headache, or any other condition described herein, comprising cooling the intranasal tissue by administration of carbon dioxide. The preferred embodiments of this aspect correspond to those described above.

Furthermore, without wishing to be bound by theory, it is believed that carbon dioxide may cause a modulation of the intracellular pH-value of nerve cells which leads to a reduction of pain. In yet another aspect, the invention therefore relates to the pressurized compositions comprising carbon dioxide as described in detail above for use in the treatment or prevention of a disorder associated with headache, or any other condition described herein, comprising modulating the intracellular pH-value of nerve cells by administration of carbon dioxide. The preferred embodiments of this aspect correspond to those described above.

Another mode of action which is believed to be involved in the effects observed by the inventors is that the carbon dioxide may have an inhibitory or antagonistic effect on mast cell degranulation. This may explain why the claimed compositions comprising carbon dioxide for use according to the present invention may be effective for treating or preventing allergic rhinitis and/or rhinosinusitis.

Within the scope of this first aspect of the invention, the present disclosure also provides for the use of carbon dioxide (CO₂) in the manufacture of a pressurized composition comprising carbon dioxide for
- the prevention or treatment of a disorder or condition associated with headache, or a disorder or condition involving the trigeminal nervous system in a subject, or
- the prevention or treatment of allergic rhinitis, rhinosinusitis, trigeminal neuralgia, post-traumatic headache, temporomandibular joint disorder, epilepsy or seizures in a subject,

wherein the composition comprising carbon dioxide is administered to the nasal cavity of the subject by intranasal administration in the form of a gaseous composition comprising carbon dioxide, and
wherein the gaseous composition comprising carbon dioxide is administered to the nasal cavity of the subject via both nostrils of the subject.

Within the scope of this first aspect of the invention, the present disclosure furthermore provides for the use of a pressurized composition comprising carbon dioxide (CO₂) for
- the prevention or treatment of a disorder or condition associated with headache, or a disorder or condition involving the trigeminal nervous system in a subject, or
- the prevention or treatment of allergic rhinitis, rhinosinusitis, trigeminal neuralgia, post-traumatic headache, temporomandibular joint disorder, epilepsy or seizures in a subject,

wherein the carbon dioxide is administered to the nasal cavity of the subject by intranasal administration in the form of a gaseous composition comprising carbon dioxide, and
wherein the gaseous composition comprising carbon dioxide is administered to the nasal cavity of the subject via both nostrils of the subject.

Furthermore, within the scope of this first aspect of the invention, the present disclosure also provides for a method for
- the prevention or treatment of a disorder or condition associated with headache, or a disorder or condition involving the trigeminal nervous system in a subject, or
- the prevention or treatment of allergic rhinitis, rhinosinusitis, trigeminal neuralgia, post-traumatic headache, temporomandibular joint disorder, epilepsy or seizures in a subject,

wherein the method comprises administration of a pressurized composition comprising carbon dioxide to the nasal cavity of the subject by intranasal administration in the form of a gaseous composition comprising carbon dioxide, and
wherein the gaseous composition comprising carbon dioxide is administered to the nasal cavity of the subject via both nostrils of the subject.

In a second aspect, the present invention provides for an applicator device for the intranasal administration of a gaseous composition comprising carbon dioxide to a subject, preferably for the intranasal administration of a composition comprising carbon dioxide for use according to the first aspect of the invention,
wherein the application device is adapted for the administration or introduction of the gaseous composition comprising carbon dioxide into the nasal cavity of the subject by intranasal administration via both nostrils of the subject, preferably via simultaneous administration via both nostrils of the subject.

To avoid repetitions, it should be noted that the entire disclosure as provided above in connection with the pressurized compositions for use according to the first aspect of the invention, including all embodiments, features limitations and combinations thereof, applies to the applicator device according to this second aspect of the invention, respectively.

Accordingly and for the avoidance of doubt, in some embodiments this second aspect of the invention also refers to an applicator device adapted for the administration or introduction of the pressurized composition, specifically of the pressurized gaseous composition comprising carbon dioxide into the nasal cavity of the subject by intranasal administration via both nostrils of the subject, preferably via simultaneous administration via both nostrils of the subject, wherein the applicator device comprises:
a) a storage unit for holding and/or providing the composition comprising carbon dioxide in pressurized form;
b) a pre-conditioning unit comprising a pre-conditioning chamber fluidically connected with the reservoir for transforming the carbon dioxide in pressurized form into the gaseous composition comprising carbon dioxide; and
c) an application unit fluidically connected with the pre-conditioning unit, specifically with the pre-conditioning chamber, for introducing the gaseous composition comprising carbon dioxide into the nasal cavity of the subject via both nostrils of the subject; and optionally
d) a housing for holding the storage unit, the pre-conditioning unit, the application unit and optionally further functional units of the applicator device.

In a third aspect, the present invention provides for a kit comprising:
- a pressurized composition comprising carbon dioxide; and
- an applicator device adapted for the intranasal administration of a gaseous composition comprising carbon dioxide to the nasal cavity of a subject via both nostrils of a subject, preferably an administration device according to the second aspect of the invention.

In connection with this aspect of the invention as well, it should be noted that the entire disclosure as provided above in connection with the compositions for use according to the first aspect of the invention, as well as in connection with the applicator device according to the second aspect of the invention including all embodiments, features limitations and combinations thereof, applies to the kits according to this third aspect of the invention, respectively.

In some embodiments of the kits according to this third aspect of the invention, the storage unit of the applicator device comprises a reservoir and wherein the reservoir is provided in the form of a separate reservoir comprising a composition comprising carbon dioxide in pressurized form.

In some embodiments, the kits according to this aspect of the invention further comprise instructions for use and/or assembly of the applicator device.

In further embodiments, the kits according to this aspect of the invention further comprise one or more plugs as described above in connection with the first aspect of the invention to be mounted on the application unit, specifically the oblong extensions of the application unit of the applicator device prior to use.

In a fourth aspect, the present invention provides for a method for the preparation of a gaseous composition comprising carbon dioxide (suitable for the administration into the nasal cavity of a subject), specifically for use according to the first aspect of the disclosure, the method comprising the steps:
- providing a pressurized composition comprising carbon dioxide, specifically a pressurized composition for use according to the first aspect of the disclosure; and
- at least partly evaporating the pressurized composition comprising carbon dioxide in pressurized form to provide a gaseous composition comprising carbon dioxide (in less pressurized form).

In some embodiments, the method according to this fourth aspect of the present invention further comprises providing an applicator device as described in detail above in connection with the second aspect of the invention. Accordingly, all it should be noted that the entire disclosure as provided above in connection with the compositions for use according to the first aspect of the invention, as well as in connection with the applicator device according to the second aspect of the invention including all embodiments, features limitations and combinations thereof, applies to the method according to this fourth aspect of the invention, respectively.

In some embodiments, the method according to this aspect of the invention further comprises the process step of:
- providing an applicator device according to the second aspect of the invention comprising a pressurized composition comprising carbon dioxide for use according to the first aspect of the invention.

In further embodiments, the method according to this aspect of the invention further comprises the process step of:
- at least partly evaporating the pressurized composition comprising carbon dioxide in pressurized form to provide a gaseous composition comprising carbon dioxide (in less pressurized form), wherein such evaporation is conducted within the applicator device.

Especially when performed using an applicator device according to the second aspect of the present invention, the present method allows for providing the gaseous composition comprising carbon dioxide at a defined temperature and/or at a defined flow rate and/or in an amount conveniently to be controlled by the user.

In yet a further aspect, the present disclosure also provides for carbon dioxide (CO₂) or the compositions comprising carbon dioxide as described in detail above for use in inhibiting mast cell degranulation or for use in the treatment or prevention of a disorder associated with, or caused by, mast cell degranulation, preferably in accordance with any one of the preceding aspects or embodiments. In connection with this further aspect, the present disclosure provides for the use of carbon dioxide (CO₂) for inhibiting mast cell degranulation. In connection with this further aspect, the present disclosure also provides for a method of treating or preventing a disorder associated with, or caused by, mast cell degranulation, comprising administering to a subject an effective amount of carbon dioxide or a composition comprising carbon dioxide as described above, preferably in accordance with any one of the preceding aspects or embodiments. In particular embodiments, said subject exhibits an elevated level of mast cell degranulation, relative to a healthy subject.

### DETAILED DESCRIPTION OF THE DRAWINGS

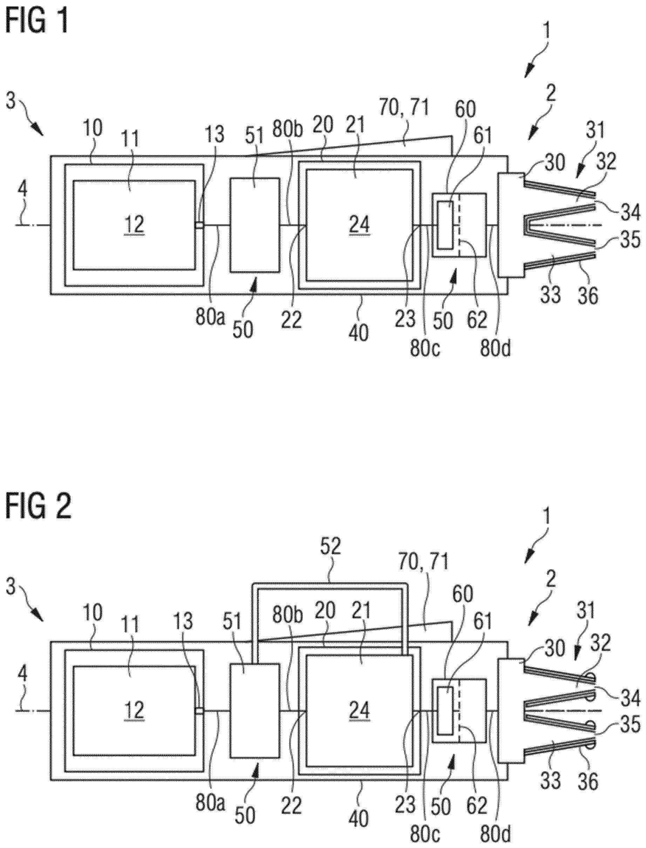

Figure 1 shows a schematic, cross-sectional, exemplary and not to scale depiction of an applicator device 1 as described in connection with the compositions comprising carbon dioxide for use according to the first aspect of the invention. The shown applicator device 1 is adapted for the administration of the gaseous composition comprising carbon dioxide into the nasal cavity of the subject by intranasal administration via both nostrils of the subject, preferably via simultaneous administration via both nostrils of the subject.

The shown applicator device 1 comprises a proximal end 2 which, in the operational mode, should be directed towards the subject, specifically towards the nostrils of the subject and a distal end 3 which is located at the opposite end which, in the operational mode, should be directed in the opposite direction, pointing away from the body or nose of the subject. The application device 1 has a main axis 4 which connects proximal end 2 with distal end 3.

The shown applicator device 1 comprises a storage unit 10 located towards the distal end 3 of the applicator device. Storage unit 10 comprises a reservoir 11 in the form of a container holding the composition comprising carbon dioxide in pressurized form 12. Reservoir 11 is connected to storage unit 10 (and consequentially to applicator device 1) via connecting unit 13 which establishes a releasable or non-releasable (depending on the replaceability of the chosen reservoir 11) fluidic connection between reservoir 11 and applicator device 1, specifically storage unit 10 of applicator device 1. Such a reservoir comprising a composition comprising carbon dioxide in pressurized form is commercially available, for example, in the form of a cylinder holding 12 g of pressurized carbon dioxide (available from Linde GmbH, Germany).

Applicator device 1 further comprises pre-conditioning unit 20 fluidically connected to storage unit 10 and located downstream of storage unit 10, i.e. in the direction of flow of the composition comprising carbon dioxide in the operational mode. Pre-conditioning unit 20 comprises pre-conditioning chamber 21 located within pre-conditioning unit 20. Preconditioning chamber 21 has an inlet 22 and an outlet 23. Inlet 22 of the pre-conditioning chamber is fluidically connected with storage unit 10 (via tubing 80a, pressure reducer 51 of control unit 50 as described further below and tubing 80b), more specifically with connecting unit 13 of storage unit 10. As can be seen in Fig. 1, pre-conditioning chamber (after release of a dose of composition 12 into the preconditioning chamber holds the gaseous composition comprising carbon dioxide 24 in pre-conditioned (i.e. less pressurized) form.

The device 1 shown in Fig. 1 further comprises application unit 30 located at the proximal end 2 of applicator device 1 and fluidically connected to pre-conditioning chamber 21 of pre-conditioning unit 20 via tubing 80d, safety and release unit 60 and tubing 80c. Application unit 30 comprises nosepiece 31 with a first 32 and a second 33 oblong extension, each oblong extension being dimensioned and shaped for the introduction into one nostril of a subject. At the outermost proximal end of the first 32 and second 33 oblong extension a first 34 and second 35 application opening is located through which the gaseous composition comprising carbon dioxide is released into the respective nostril of the subject. On the outer surface of first 32 and second 33 oblong extension a plug 36 is provided, here in the optional form of one single unit for both oblong extensions 32, 33. Such plug may be useful for the tight closure of the nostrils of the subject once the oblong extensions 32, 33 are introduced into the nostrils and may be provided, as described in detail above, in the form of a deformable, elastic material.

Fig. 1 further shows the optional, however, preferred housing 40 holding i.a. storage unit, 10, pre-conditioning unit 20 and application unit 30 (at least partly). The housing, as described above, may be dimensioned and shaped to hold the functional units comprised by the present device 1 while being holdable and by one or both hands of a subject or user when in use. Housing 40 may have an overall longitudinal shape with a proximal and a distal end roughly corresponding to the proximal 2 and distal 3 end of device 1. Fig. 1 also schematically depicts control unit 50 comprising reservoir expansion valve or pressure reducer 51 located between (and fluidically connected to) storage unit 10 and preconditioning unit 20. Such a reservoir expansion valve 51 is commercially available, for example, in the form of pressure reducer, for example LIV Valve (available from Linde GmbH, Germany).

Control unit 50 comprises safety and release unit 60 comprising valve 61 and unidirectional membrane 62 located between (and fluidically connected to) preconditioning unit 20 and application unit 30 via tubings 80c and 80d, respectively. Fig. 1 further schematically depicts an actuation unit 70 with push button 71 arranged within and accessible from outside of housing 40.

Figure 2 schematically depicts a further embodiment of applicator device 1 in which control unit 50, specifically reservoir expansion valve 51, is fluidically connected to the (inner volume of) pre-conditioning chamber 21 of pre-conditioning unit 20 via dosage regulator channel 52. This dosage regulator channel 52 allows for the partial backflow of the (expanded) gaseous composition comprising carbon dioxide 24 as formed in preconditioning chamber 21 towards reservoir expansion valve 51 and, accordingly, allows for the closure of reservoir expansion valve 51 once a defined pressure has been reached in pre-conditioning chamber 21, for example about 5 bar. This, furthermore, allows for the exact and reproduceable dosage of the gaseous composition comprising carbon dioxide to be released from nosepiece 31 of application unit 30 into the nostrils of the subject.

In Fig. 2, furthermore, an alternative embodiment of plug 36 is shown in which plug 36 is provided in a form comprising two separate plugs to be (releasably) mounted on each oblong extension 32, 33 of nosepiece 31 wherein each plug has enlargements at the proximal end. All additional features as shown in Fig. 2 correspond to those as described in connection with Fig. 1 above and are not described again in order to avoid repetitions.

### List of reference numerals:

- 1: Applicator device
- 2: Proximal end of applicator device
- 3: Distal end of applicator device
- 4: Main axis of applicator device
- 10: Storage unit
- 11: Reservoir (or container)
- 12: Composition comprising carbon dioxide in pressurized form
- 13: Connecting unit (connecting port)
- 20: Pre-conditioning unit
- 21: Pre-conditioning chamber
- 22: Inlet (of pre-conditioning chamber)
- 23: Outlet (of pre-conditioning chamber)
- 24: Gaseous composition comprising carbon dioxide
- 30: Application unit
- 31: Nosepiece of the application unit
- 32: First oblong extension
- 33: Second oblong extension
- 34: First application opening
- 35: Second application opening
- 36: Plug
- 40: Housing
- 50: Control unit
- 51: Reservoir expansion valve; pressure reducer
- 52: Dosage regulator channel
- 60: Safety and release unit
- 61: Valve (of safety and release unit)
- 62: Unidirectional membrane
- 70: Actuation unit
- 71: Push button (of actuation unit)
- 80: Tubing (a to d)

The following examples serve to illustrate the invention without, however, restricting the scope of the invention in any regard:

### EXAMPLES

### Example 1: Intranasal administration of a pressurized composition comprising carbon dioxide simultaneously via both nostrils

An experimental applicator device suitable for the intranasal administration of a pressurized gaseous composition consisting of carbon dioxide by simultaneous introduction of the composition into both nostrils of a human individual was provided. The device comprised a container with an inner volume of 500 mL containing 495 g of pure CO₂ (with a purity of 98 % at a pressure of about 50 bar resulting in an extractable volume of CO₂ of about 60 L.

The container was fluidically connected via silicon tubing with a length of 10 cm and a diameter of 1.3 mm to the inlet opening of a hollow chamber having in inner volume of 11 mL (pre-conditioning chamber) via a Sigma 2SSH-MA-VM6 pressure reducer (available e.g. from Linde GmbH, Germany) configured to reduce the pressure of carbon dioxide within the pre-conditioning chamber at a defined value within the range of from about 2 to 6 bar (specifically at 2, 4 and 6 bar). The pre-conditioning chamber had an outlet opening which was fluidically connected to a silicon nosepiece from Nizirioo with two outlet openings in the form of two nasal cannulas suitable for the simultaneous introduction into one nostril of the individual via a tubing with a length of 50 cm and a diameter 1.3 mm. For providing a gas-tight connection between each nostril and the corresponding outlet opening of the nosepiece, on each outlet opening a foam sealing plug was provided. Accordingly, the test persons were not able to breathe in or out through the nose during administration.

Using this experimental applicator device, carbon dioxide in gaseous form was administered intranasally to a test group of three voluntary adult test persons (one female, two male) who were in general in good health. The outlet openings of the nosepiece fitted with corresponding sealing plugs were introduced into both of the corresponding individual's nostrils and carbon dioxide was administered simultaneously intranasally in sitting position at a pressure of CO₂ within the pre-conditioning chamber of 2 bar, 4 bar and 6 bar, respectively for a period of 10 s or 20 s each.

The amount of CO₂ administered to the nasal cavity of a test person at a specific pressure (within the pre-conditioning chamber) and for a specific time (either 10 s or 20 s) are summarized in Table 1 below:

**Table 1: Amounts of CO₂ administered intranasally:**

| Pressure¹ (bar) | Administration time (s) | Amount of CO₂ (mL) | Amount of CO₂ (mL/sec) |
|---|---|---|---|
| 2 | 10 | 90 | 9 |
| 2 | 20 | 180 | 9 |
| 4 | 10 | 180 | 18 |
| 4 | 20 | 360 | 18 |
| 6 | 10 | 360 | 36 |
| 6 | 20 | 720 | 36 |

The following parameters were measured:
- the effective nasal flow rates of carbon dioxide;
- the maximum tolerable nasal flow rates;
- the temperature at the outlet openings of the nosepiece (during administration of CO₂ into nostrils (°C);
- the onset of sensation (e.g. tingling in the nose) (s); and
- the onset of other perceptions (e.g. pain) (s).

When CO₂ was administered at a pressure of 2 bar or 4 bar, respectively, a temperature of 10°C (at a room temperature of 24 °C) was measured within the nostrils at the outlet opening of the nosepiece in all cases. This temperature was measured for the first 3 s after start of the administration and afterwards quickly adapted to the temperature of the nasal environment. Furthermore, when administered at a pressure of 4 bar an effective nasal flow rate of carbon dioxide of 18 mL/s was determined.

When CO₂ was administered at a pressure of 6 bar, a temperature of 8°C (at a room temperature of 24 °C) was measured within the nostrils at the outlet opening of the nosepiece in all cases. This temperature was measured for the first 5 s after start of the administration and afterwards quickly adapted to the temperature of the nasal environment. Furthermore, when administered at a pressure of 6 bar an effective nasal flow rate of carbon dioxide of 36 mL/s was determined.

The further experimental results as obtained above for the individual test persons are summarized in Tables 2 to 4 below.

**Table 2: Results of experimental intranasal administration of pressurized CO₂ for test person 1 (female):**

| Pressure ¹ (bar) | Administration time (s) | Onset of sensatio n (s) | Onset of nasal tingling (s) | Onset of numbness (s) | Tolerability | Onset of cooling sensation (s) / duration (s) |
|---|---|---|---|---|---|---|
| 2 | 10 | 2 | 3 | | Comfortable | 2 / 3 |
| 2 | 20 | 2 | 3 | | Comfortable | 2 / 3 |
| 4 | 10 | 1-2 | 2 | | Comfortable | 2-3 / 2-4² |
| 4 | 20 | 1-2 | 2 | 14 | Tolerable | 2-3 / 2-4 |
| 6 | 10 | 1 | 1-2 | | Tolerable | 2 / 2-6 |
| 6 | 20 | 1 | 1-2 | 8 | Uncomfortab le | 2 / 2-6 |

| | | | | | | |
|---|---|---|---|---|---|---|
| ¹ within pre-conditioning chamber ² Cooling sensation after 2 to 3 s which lasted for 2 to 4 s | | | | | | |

Furthermore, when CO₂ was administered at 6 bar this test person reported the onset of pain after 20 s of administration. Furthermore, palate tingling was reported upon CO₂ administration at 4 bar after 15 s and at 6 bar after 11 s.

**Table 3: Results of experimental intranasal administration of pressurized CO₂ for test person 2 (male):**

| Pressure' (bar) | Administration time (s) | Onset of sensation (s) | Onset of nasal tingling (s) | Onset of numbness (s) | Tolerability | Onset of cooling sensation (s) / duration (s) |
|---|---|---|---|---|---|---|
| 2 | 10 | 2 | 8 | | Comfortable | 3 / 3 |
| 2 | 20 | 2 | 8 | | Comfortable | 3 / 3 |
| 4 | 10 | 2 | 6 | | Comfortable | 2-3 / 2-4 |
| 4 | 20 | 2 | 6 | | Comfortable | 2-3 / 2-4 |
| 6 | 10 | 1 | 4 | | Tolerable | 2 / 2-6 |
| 6 | 20 | 1 | 4 | 10 | Tolerable | 2 / 2-6 |

| | | | | | | |
|---|---|---|---|---|---|---|
| ¹ within pre-conditioning chamber | | | | | | |

Furthermore, when CO₂ was administered at 6 bars for 20 s this test person reported the onset of pain after 18 s of administration. Furthermore, palate tingling was reported upon CO₂ administration at 4 bar after 12 s and at 6 bar after 10 s.

**Table 4: Results of experimental intranasal administration of pressurized CO₂ for test person 3 (male):**

| Pressure ¹ (bar) | Administration time (s) | Onset of sensation (s) | Onset of nasal tingling (s) | Onset of numbnes s (s) | Tolerability | Onset of cooling sensation (s) / duration (s) |
|---|---|---|---|---|---|---|
| 2 | 10 | 2-3 | 5 | | Comfortable | 3 / 3 |
| 2 | 20 | 2-3 | 5 | | Comfortable | 3 / 3 |
| 4 | 10 | 1-2 | 4-5 | | Comfortable | 2-3 / 2-4 |
| 4 | 20 | 1-2 | 4-5 | | Comfortable | 2-3 / 2-4 |
| 6 | 10 | 1 | 3 | 9 | Comfortable | 2 / 2-6 |
| 6 | 20 | 1 | 3 | 9 | Uncomfortab le | 2 / 2-6 |

| | | | | | | |
|---|---|---|---|---|---|---|
| ¹ within pre-conditioning chamber | | | | | | |

Furthermore, when CO₂ was administered at 6 bars for 20 s this test person reported the onset of pain after 20 s of administration. Furthermore, palate tingling was reported upon CO₂ administration at 6 bar after 8 s.

These examples have shown that under pressures within the range of from 2 to 6 bar, specifically ranges from 4 bar to 6 bar via both nostrils simultaneously and nostrils sealed, CO₂ application to the nasal cavity was well tolerated.

### Example 2: Investigation of the effect of CO₂ on intracellular pH, Hsp70 (heat shock protein 70) expression, and calcium ion channel activity in primary cultures of trigeminal ganglion neurons

In order to investigate the conditions inducing a physiological reaction upon application of a gaseous composition comprising CO₂, an experimental *in-vitro* test setting using an established model for preparation, cultivation and investigation of primary cultures of trigeminal ganglion neurons was used. Several of the standard conditions, however, were adapted in order to mimic the situation as given during intranasal administration of a gaseous composition comprising CO₂ via both nostrils into the human nasal cavity achieving an almost 100% saturation with CO₂, namely to bring the composition comprising CO₂ next to the trigeminus ganglion nerve endings in the nasal cavity, to apply the composition comprising CO₂ in a distinct amount, and to apply the composition comprising CO₂ over a very short period of time. As indicators for the physiological reaction caused by the application of the composition comprising CO₂ the intracellular pH, hsp70 expression and calcium ion channel expression in primary cultures of the trigeminal ganglion were investigated.

### 2.1 Materials and Methods

Animals: Male and female neonatal Sprague-Dawley 3-5-day old pups were utilized for establishing primary cultures of the trigeminal ganglion. All experiments were approved by the Institutional Animal Care and Use Committee at Missouri State University, and conducted in accordance with institutional, ARRIVE, and NIH guidelines (Protocol 2022-08 approved on April 29, 2022; Animal Welfare Assurance Number D16-00033). A concerted effort was made to minimize the number of animals used for this study. Female pregnant adult Sprague-Dawley rats were purchased from Missouri State University's internal breeding colony (Springfield, MO). Animals were housed in clear, plastic cages with unrestricted access to food and water. Animal holding rooms were on a 12-hour light/dark cycle and maintained at a constant temperature of 22-24°C. The animal's overall health was monitored daily by vivarium staff.

Establishing Primary Cultures of Trigeminal Ganglia: Trigeminal ganglia were obtained from neonatal 3-5-day old rat pups and placed in 4°C HEPES buffered L-15 media (pH 7.4), referred to as plating media, during the duration of dissections. Following dissections, tissues were placed in room temperature plating media with Dispase II (Sigma Aldrich, St. Louis, MO) and RQ1 DNase (Promega, Madison, WI), and incubated at 37°C for 30 minutes while being constantly rotated at 15 RPM (revolutions per minute) to enzymatically digest connective tissue and promote cell dissociation. Following incubation, tissues were centrifuged at 500 RPM for 2 min, the resulting supernatant was decanted, and tissues resuspended in 5 mL of plating media. Vigorous mechanical trituration was performed to further dissociate the tissues. The supernatant containing cells were transferred to a sterile 15 mL tube, and the dissociation step was repeated. The final 10 mL of cell-containing supernatant was centrifuged at 1300 RPM for 3 min to pellet dissociated neurons and glial cells. For cryopreservation, cells were resuspended in a 37°C solution of 90% fetal bovine serum (FBS, Atlanta Biologicals, Norcross, GA), and 10% dimethyl sulfoxide (DMSO, Sigma), and aliquoted into cryogenic vials for storage. Vials were slowly frozen using a freezing container (Mr. Frosty, ThermoFisher Scientific, Waltham, MA) placed inside of a -80°C freezer overnight. The following day, vials were quickly transferred to a liquid nitrogen Dewar where they remained in long-term storage for up to one year. For plating of fresh cell culture preparations, the cell pellet was resuspended in a 37°C L-15 medium containing 10% FBS (Atlanta Biologicals), 50 mM glucose (Sigma-Aldrich), 250 mM ascorbic acid (Sigma-Aldrich), 8 mM glutathione (Sigma-Aldrich), 2 mM glutamine (Sigma-Aldrich), 10 ng/mL mouse 2.5 S nerve growth factor (Alomone Laboratories, Jerusalem, Israel), an antibiotic mixture of penicillin (100 units/mL) and streptomycin (100 µg/mL, Sigma-Aldrich), and the antimycotic amphotericin B (2.5 mg/mL, Sigma-Aldrich). The resulting media is herein referred to as "TG complete medium". To begin an experiment using cryopreserved cells, vials of frozen cells were gradually thawed by addition of 37°C TG complete medium. One vial consisting of 0.5 mL of cryopreservation solution was thawed in 5-10 mL of warm TG complete medium to immediately dilute the DMSO, to minimize the loss of cell viability. For immunocytochemistry, intracellular pH measurements, and calcium imaging, fresh or cryopreserved cells were plated on 12 mm poly-L-lysine coated glass coverslips (Electron Microscopy Sciences, Hatfield, PA) at a density of about 1 ganglion (~34,000 cells) per 24-well plate (Greiner Bio-One, Monroe, NC) in 500 µl of TG complete medium per well. Cultures for all experiments were left to incubate at 37°C in a humidified chamber at ambient CO₂ levels. Cells were observed daily using a light microscope to look for any abnormal cell morphologies and/or low cell density, and changes in the media pH were monitored via the color indicator present in the L-15 media.

CO₂ Administration: For the CO₂ exposure studies on trigeminal ganglion cells and all intracellular measurements, cultures plated and maintained on coverslips were removed from the 24-well plate and covered with the exact volume of HBS media that was used for the external pH measurements, and then placed in a saturated CO₂ chamber for varying amounts of time. The amount of medium was the minimal amount required to cover the cells and prevent desiccation during the experiment. Compressed pure CO₂ was administered into a chamber containing cultured trigeminal ganglion neurons being maintained in a viable state in a HEPES-buffered saline solution, pH 7.2. The chamber consisted of a rectangular box with unobstructed flow of CO2 achieving an almost saturated CO2 atmosphere in the chamber. Cells were covered with a very thin film of buffered saline to simulate humidified conditions in the nasal/sinus cavities.

Statistics: Each experimental condition was performed in duplicate and repeated in at least 3 independent experiments. The levels of hsp70 were reported as the average relative staining intensity ± SEM after values have been normalized to basal levels. Results of intracellular Ca²⁺ and pH imaging were shown relative to basal levels under similar culture conditions. JASP version 0.17.2.1 was used to evaluate the differences in fluorescent protein expression between conditions. Due to the data being an N of less than 20, a non-parametric Kruskal-Wallis test was used to determine if significant differences existed between all groups. If P < 0.05 in the Kruskal-Wallis test, a Dunn post-hoc test was used for pairwise comparison. Differences were considered significant if P < 0.05.

### 2.2 Extracellular pH-Measurements:

The effect of increasing the concentration of CO₂ to saturation (100%) on the pH of an unbuffered HBS medium was determined. HBS medium at a pH of 7.4 was added to coverslips (80 µl), placed in a saturated CO₂ chamber and removed after 10, 20, 30, and 60 s of CO₂ exposure and the pH of the media measured using pH strips and a pH meter to determine changes in the external pH. With this setting it was intended to mimic the in vivo situation where the media (nasal fluid) would not be strongly buffered.

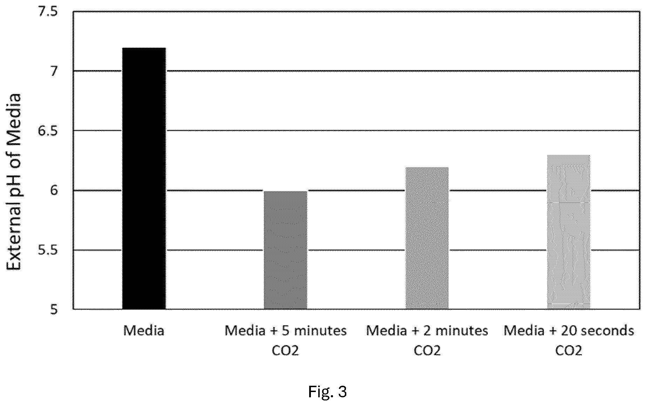

Results: When compared to (unexposed) media alone (first column from the left), the pH of the media became acidic when exposed to CO₂ with a slightly lower pH observed for an exposure of 5 min (6.0; second column from the left) when compared to an exposure of 2 min (6.2; third column from the left) and 20 s (6.3; fourth column from the left). The pH dropped very rapidly. These results are depicted in Fig. 3.

### 2.3 Intracellular pH-Measurements:

For intracellular pH-imaging, cells were plated in HBS media at a density as described above on poly-L-lysine coated, 11 mm glass coverslips in a 24-well plate. Cells were incubated with pH-sensitive loading dyes, following manufacturer's instructions (ThermoFisher Scientific): pHrodo Red AM and pHrodo Green AM indicator dyes (Thermo Fisher Scientific) were added following the manufacturer's instructions to the culture media and incubated at 37°C for 30 min. Fresh medium was used to rinse the cells once and the plate will be incubated for another 30 min. Individual coverslips were imaged using a fluorescent microscope.

Initially, the relative change in intracellular pH (pHᵢ) was determined in cultured cells in response to incubation in unbuffered media exposed for 5 min or 20 s to a gaseous composition comprising 100% CO₂. The Invitrogen Intracellular pH Calibration Buffer Kit was used as directed by the manufacturer to normalize the data (ThermoFisher Scientific). For experiments designed to measure intracellular pH changes in response to CO₂ exposure, coverslips were placed in a sealed chamber saturated with 100% CO₂ for 5 min or 20 s prior to pH measurement. Data were reported as the ratio of the wavelength measurements. Each experimental condition was repeated a minimum of three times.

Results: The exposure of the cultured trigeminal ganglion neuronal cells to a gaseous composition comprising 100% CO₂ for 5 min and even 20 s caused a change in intracellular pH in both Aδ- and C-fiber neurons at 37° C. A change in intracellular pH from the normal 7.2-7.4 range is stressful to cells and will suppress normal enzyme function and hence, inhibit the synthesis and release of inflammatory molecules from sensory neurons.

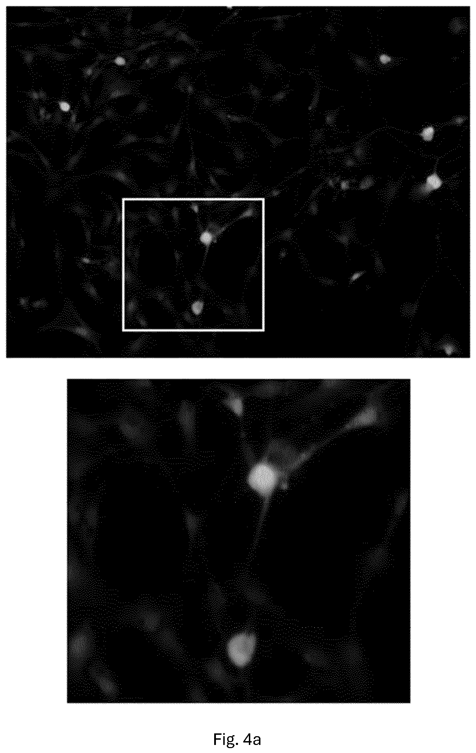

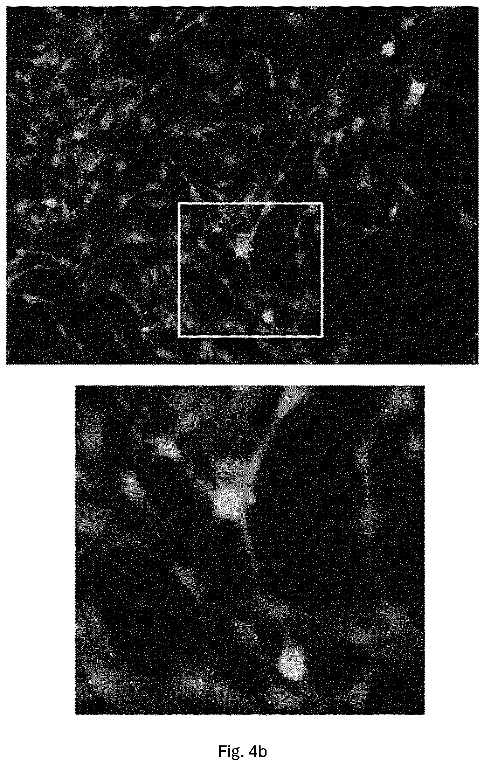

Figs. 4a and 4b depict the results of the administration of CO₂ for 20 s to trigeminal ganglion neuronal cells caused changes in the intracellular pH levels. Relative change in intracellular pH was determined using a fluorescent dye that becomes brighter with lower intracellular pH levels. The lower picture in both Figures shows an enlargement of the section as framed in the corresponding upper picture. Fig. 4a depicts cultures of trigeminal ganglion neurons left untreated (naive, N, i.e. not exposed to CO₂) while Fig. 4b depicts cultures of trigeminal ganglion neurons subjected to 20 s of CO₂ administration. When compared to naive untreated cells (as shown in Fig 4a), the intensity of fluorescence staining was much greater in cells exposed to CO₂ for 20 s (as shown in Fig. 4b), which demonstrates a decrease in intracellular pH (more acidic) that was observed in neurons (large round cells).

### 2.4 Immunocytochemistry: Hsp70 protein

Heat shock protein 70 (Hsp70) is a molecular chaperone protein that is expressed in response to stress. In this role, Hsp70 binds to its protein substrates and stabilizes them against denaturation or aggregation until conditions improve. Changes in the expression of Hsp70 protein in primary trigeminal ganglion cultures exposed to CO₂ were investigated using immunocytochemistry. Trigeminal ganglion cells were plated in HBS media at a density as described above on poly-L-lysine coated, 11 mm glass coverslips in a 24-well plate. Cells were exposed to 100% CO₂ for 5 min or 20 s prior to fixation via incubation for 15 minutes in a 4% paraformaldehyde solution diluted in PBS. Cells were incubated for 20 min with a PBS solution containing 0.1% Triton X-100 (Sigma Aldrich) and 5% donkey serum (Jackson ImmunoResearch Laboratories, West Grove, PA) to block and permeabilize the cells. Cells were then incubated with primary antibodies including a monoclonal antibody to Hsp70 diluted in 1% donkey serum and incubated overnight at 4°C in a humidified chamber. Cells were incubated with Alexa-Fluor conjugated secondary antibodies (Jackson ImmunoResearch Laboratories) diluted 1:200 in PBS for 1 hour at room temperature. Coverslips were rinsed with PBS prior to being placed on Superfrost Plus glass microscope slides (ThermoFisher Scientific). Vectashield anti-fade mounting medium containing the dye DAPI was added to each coverslip to stain the nucleus of each cell and to preserve the fluorescent signal in the cells. A rectangular glass coverslip was placed over each slide and secured using a clear coat of nail polish. Slides were stored at 4°C until imaging. Images were taken at 200x magnification using a Zeiss Axiocam mRm camera mounted on a Zeiss Imager Z1 fluorescent microscope to capture images of fluorescent staining as well as to identify cell morphology with DIC images. As a control, some slides were incubated with only secondary antibodies to confirm the specificity of the antibodies.

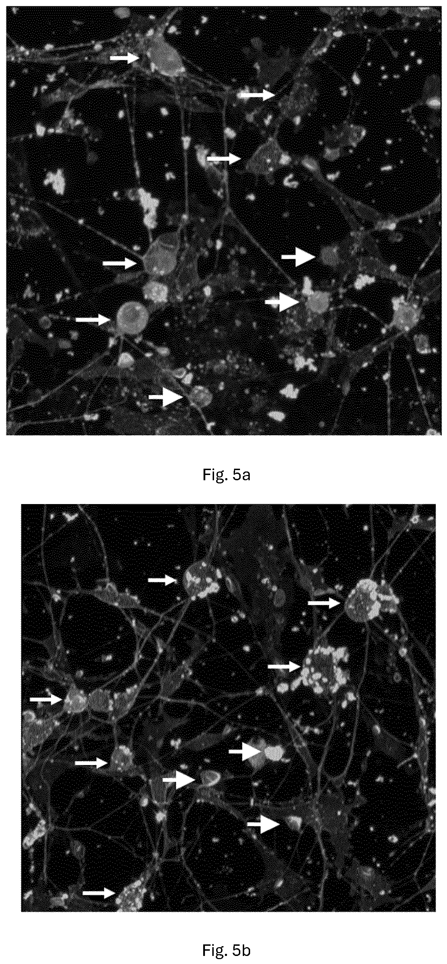

Figs 5a and 5b show the observed changes in Hsp70 expression in trigeminal ganglion neuronal cells after exposure to a gaseous composition comprising CO₂. Changes in Hsp70 expression were investigated using immunocytochemistry where the bright signals as highlighted by the arrows in Figs 5a and 5b correspond to localization of the Hsp70 protein inside neuronal cells. An exposure of neuronal cells to CO₂ for 5 min or 20 s, respectively, caused an increase in the expression of Hsp70 and a change in its cellular distribution.

Cultures of trigeminal ganglion neurons were left untreated (naive, N, see Fig. 5a) or subjected to 20 s of CO₂ administration (see Fig. 5b). After CO₂ administration (see Fig. 5b), Hsp70 was observed clustered in the nucleus and concentrated under the plasma membrane of Aδ-(small arrows) and C-fiber neuronal cells (large arrows) while in non-treated naive cells (see Fig. 5a) Hsp70 was more uniformly distributed throughout the cytoplasm of both types of neurons. The results show that Hsp70 expression changes are consistent with inhibition of cellular activities (in both A and C neurons). Hsp70 functions as an anti-inflammatory protein by inhibiting the synthesis and release of pro-inflammatory molecules.

### 2.5 Calcium Imaging:

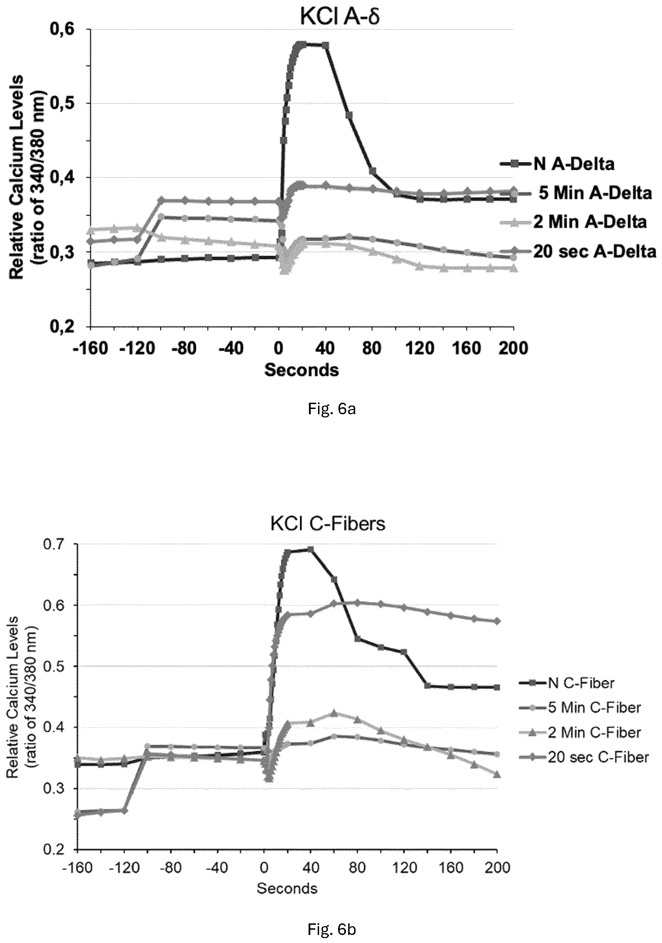

For intracellular calcium imaging, plating conditions were identical to those used for the pH imaging and Hsp70 studies. To determine the effect of CO₂ exposure to trigeminal ganglion neurons, intracellular calcium levels was determined under basal conditions (unstimulated or treated) and in response to stimulation with 60 mM potassium chloride (KCl) using the ratiometric fluorescent dye Fura-2 essentially as previously described (C. Vause et al.;Headache. 2007; 47(10): 1385-1397). Cryopreserved primary cultures were plated on poly-L-lysine coated glass coverslips at a density of approximately 1 trigeminal ganglion per 24-well plate and incubated overnight at 37 °C. The following day cells were rinsed with a standard HEPES-buffered saline (HBS, pH 7.4) solution two times, and then 500 µL of HBS was added to each well. Fura-2 AM ester (ThermoFisher Scientific) stock solution at a concentration of 1 mM was added to the well at 1:1000 for a final concentration of 1 µM in the well and cells incubated for 30 min at 37°C for loading of the Fura-2 dye. After incubation, cells were rinsed twice with HBS and then incubated for 30 min at 37°C before initiating calcium measurements. Using an Olympus IX83 microscope and Olympus CellSens Dimensions software version 4.1 (Evident Scientific, Tokyo, Japan) neurons and glial cells were clearly identified by their unique morphologies and size differences. Inside the 37°C heated and humidified chamber, absorbance was measured at 340 and 380 nm every 10 s for 120 s to establish baseline measurements. After baseline measurements for 120 s, 3 M KCl was added at a 1:50 dilution to the HBS for a final concentration of 60 mM, and ratiometric absorbance measurements immediately continued. After the addition of KCl, the change in absorbance was measured once every second for 10 s and then switched to measurements taken every 20 s for 280 s, for a total measurement time of 300 s after stimulation. The resulting data was summarized in Figs. 6a and 6b as graphs of the average ratio of F340/F380 nm wavelength values that correspond to bound and unbound intracellular calcium levels in C-fiber neurons and Aδ-fiber neurons. Each experiment was performed a minimum of 3 times.

To determine the effect of 100% CO₂ on the KCl-stimulated change in neuronal intracellular calcium, cultures were established as described above. Cells on a coverslip were placed in the chamber and then exposed for 5 min, 2 min or 20 s to CO₂. The cells were then transferred to the microscope stage and basal intracellular calcium levels measured for 2 min prior to addition of KCl to the medium. The measurements were performed at 37° C to simulate the normal human condition, and measurements were collected at the same timepoints as described.

The experiment revealed that administration of CO₂ resulted in full suppression of KCL stimulated increases in intracellular calcium levels in Aδ- and C-fiber neurons. CO₂ administration for 20 s caused a change in the normal response Aδ- and C-fiber neurons. CO₂ mediated a sustained elevation of intracellular calcium. Both types of responses to CO₂ are associated with inhibition of neuronal cell activity. A-fibers are responsible for conduction of fast, sharp, intense pain signals while C-fibers are responsible for more prolonged dull aching pain signaling. A-fibers send an initial warning of danger while C-fibers remain active to promote an inflammatory response and keep the tissue in a sensitized, protected state.

Figs. 6a and 6b summarize the changes in the intracellular level of calcium caused by the administration of CO₂ for 20 s to trigeminal ganglion neuronal cells. The x-axis depicts time in seconds. KCl was added at time zero. Cultures of trigeminal ganglion neurons were left untreated (naive, "N") or treated with 60 mM KCl, which resulted in a transient elevation of intracellular calcium. Fig. 6a depicts the results of CO₂ delivered to cells for 5 min, 2 min, or 20 s that suppressed calcium levels in Aδ-neurons when compared to untreated naive cells. Fig. 6b depicts the results of CO₂ administration to C-fibers for 5 min or 2 min resulting in suppressed calcium levels while an administration for 20 s resulted in a sustained elevated level of calcium that differed from the naive condition in which calcium levels were not sustained. A sustained calcium level increase blocks the stimulated release of CGRP. This type of elevated calcium response has been reported for the anti-migraine drug sumatriptan and shown to correlate with inhibition of trigeminal neuron activation.

The following is a list of embodiments E1 to E52 comprised by the present invention without, however, restricting it in any respect:
E1. A pressurized composition comprising carbon dioxide (CO₂) for use
   - in the treatment or prevention of a disorder or condition associated with headache, or a disorder or condition involving the trigeminal nervous system in a subject, or
   - in the treatment or prevention of allergic rhinitis, rhinosinusitis, trigeminal neuralgia, post-traumatic headache, temporomandibular jointdisorder, epilepsy or seizures in a subject,

   wherein the composition comprising carbon dioxide is administered to the nasal cavity of the subject by intranasal administration in the form of a gaseous composition comprising carbon dioxide, and
   wherein the gaseous composition comprising carbon dioxide is administered to the nasal cavity of the subject via both nostrils of the subject.
E2. The composition for use according to embodiment E1, wherein the pressurized composition comprising carbon dioxide comprises carbon dioxide in a concentration of at least 50 mol-% or of at least 75 mol-% or of at least 90 mol-% (with regard to the total composition).
E3. The composition for use according to embodiment E1 or E2, wherein the gaseous composition comprising carbon dioxide is administered in the form of a first dose administered within a treatment period of 10 min.
E4. The composition for use according to any one of the preceding embodiments, wherein the gaseous composition is administered in the form of a sole dose, specifically in the form of a sole dose administered within the treatment period of 10 minutes.
E5. The composition for use according to any one of the preceding embodiments, wherein the gaseous composition is administered in the form of a metered dose.
E6. The composition for use according to any one of the preceding embodiments, wherein the gaseous composition is administered in the form of at least one discrete dose.
E7. The composition for use according to any one of the preceding embodiments, wherein the gaseous composition comprising carbon dioxide is administered to the nasal cavity of the subject in an amount of or wherein a dose of the gaseous composition has a volume selected within the range of from about 1 mL to about 1200 mL, or of from about 5 mL to about 600 mL, or from about 10 mL to about 500 mL, or from about 10 mLto about 400 mL or of from about 20 mLto about 300 mL.
E8. The composition for use according to any one of the preceding embodiments, wherein the intranasal administration comprises exposing (contacting) at least a part of the nasal mucosa of the subject with the gaseous composition comprising carbon dioxide for a duration of from about 1 s to about 120 s or to about 90 s, or of from about 1 s to about 60 s.
E9. The composition for use according to any one of the preceding embodiments, wherein during administration of the gaseous composition comprising carbon dioxide the subject does not inhale (breathe)/refrains from inhaling/breathing, specifically, wherein during administration of the gaseous composition comprising carbon dioxide the subject does not inhale (breathe)/refrains from inhaling/breathing through the nose.
E10. The composition for use according to any one of the preceding embodiments in the treatment or prevention of a disorder or condition associated with headache or a disorder or condition involving the trigeminal nervous system in a subject.
E11. The composition for use according to any one of the preceding embodiments, wherein the disorder or condition or condition associated with headache is migraine.
E12. The composition for use according to embodiment E11, wherein the disorder or condition associated with headache is migraine without aura or migraine with aura.
E13. The composition for use according any one of the preceding embodiments, wherein the subject is a human subject.
E14. The composition for use according to embodiment E12, wherein the subject, preferably the human subject, does not respond or poorly responds to the treatment with triptans.
E15. The composition for use according to any one of the preceding embodiments, wherein the gaseous composition is administered to the nasal cavity simultaneously via both nostrils of the subject.
E16. The composition for use according to any one of the preceding embodiments, wherein the concentration of carbon dioxide in the gaseous composition is selected within the range of from about 80 mol-% to about 99,99 mol-%.
E17. The composition for use according to any one of the preceding embodiments, wherein the gaseous composition is administered in preconditioned form.
E18. The composition for use according to any one of the preceding embodiments, wherein the gaseous composition is administered to the nasal cavity of the subject at a temperature within the range of from about 0 °C to about 18 °C or of from about 5 °C to about 15 °C (as measured at the exit of the device during the first second of the initiation of the application).
E19. The composition for use according to any one of the preceding embodiments, wherein the gaseous composition is administered to the nasal cavity of the subject at a pressure within the range of from about 1.5 bar to about 10 bar or of from about 4 bar to about 6 bar.
E20. The composition for use according to any one of the preceding embodiments, wherein the gaseous composition is administered to the nasal cavity of the subject at a flow rate of from about 1 mL/s to about 30 mL/s, or of from about 5 mL/s, or of from about 10 mL/s to about 25 mL/s or to about 20 mL/s.
E21. The composition for use according to any one of the preceding embodiments, a discrete and/or metered dose of the gaseous composition comprising carbon dioxide comprises carbon dioxide in an amount of from about 20 mg to about 700 mg, or of from about 45 mg to about 530 mg or of from about 70 mg or of from about 85 mg to about 400 mg, or of from about 85 mg to about 350 mg, whereas the remaining gaseous constituents may be air or other physiologically and/or pharmaceutically acceptable gases.
E22. The composition for use according to any one of the preceding embodiments, wherein the gaseous composition is to be administered to the nasal cavity of the subject within a period of from about 1 s to about 30 s, or of from about 3 s or from about 4 s to about 25 s or to about 20 s, or of from about 5 s to about 15 s or from about 5 s to about 12 s (as measured from the beginning to the end of the administration of the gaseous composition into the nasal cavity of the subject).
E23. The composition for use according to any one of the preceding embodiments, wherein the gaseous composition is administered using an applicator device (1).
E24. The composition for use according to embodiment E23, wherein the applicator device (1) is adapted to generate an atmosphere enriched with carbon dioxide in the nasal cavity of the subject, specifically adapted to generate an atmosphere comprising a concentration of carbon dioxide within the range of from about 70 mol-% to about 99 mol-%, or from about 80 mol-% to about 99.9 mol-%.
E25. The composition for use according to embodiments E23 or E24, wherein the applicator device (1) comprises:
   a) a storage unit (10) for holding and/or providing the composition comprising carbon dioxide in pressurized form (12);
   b) a pre-conditioning unit (20) comprising a pre-conditioning chamber (21) fluidically connected with the storage unit for transforming the composition comprising carbon dioxide in pressurized form (12) into a gaseous composition comprising carbon dioxide (24); and
   c) an application unit (30) fluidically connected with the pre-conditioning unit (20), specifically with the pre-conditioning chamber (21), for introducing the gaseous composition comprising carbon dioxide (24) into the nasal cavity of the subject via both nostrils of the subject; and optionally
   d) a housing (40) for holding the storage unit (10), the pre-conditioning unit (20), the application unit (30) and optionally further functional units of the applicator device (1).
E26. The composition for use according to embodiment E25, wherein the storage unit (10) comprises a reservoir (11) and wherein the reservoir comprises the carbon dioxide (12) in (essentially) pure form or in the form of a mixture comprising carbon dioxide in an amount of at least about 50 mol-% or of at least about 75 mol-% with regard to the total amount of such mixture.
E27. The composition for use according to embodiment E26, wherein the reservoir (11) comprises the carbon dioxide (12) in condensed form (in the form of a liquid).
E28. The composition for use according to any one of embodiments E25 to E27, wherein the reservoir (11) comprises the composition comprising carbon dioxide (12) at a pressure of from about 40 bar to about 60 bar.
E29. Carbon dioxide for use according to any one of embodiments E25 to E28, wherein the reservoir (11) has a volume of from about 1 mL to about 500 mL, or of from about 1 mL to about 250 mL or to about 150 mL.
E30. The composition for use according to any one of embodiments E25 to E29, wherein the application device (1) comprises a replaceable reservoir (11).
E31. The composition for use according to any one of embodiments E25 to E29, wherein the application device (1) comprises a non-replaceable reservoir (11).
E32. The composition for use according to any one of embodiments E25 to E31, wherein the pre-conditioning chamber (21) has an inner volume of from about 1 mL to about 10 mL or from about 2 mL to about 6 mL.
E33. The composition for use according to any one of embodiments E25 to E32, wherein the application device (1) further comprises:
   e) a control unit 50) for controlling at least one of the amount, pressure, temperature and flow rate at which the gaseous composition comprising carbon dioxide (24) is administered to the nasal cavity of the subject.
E34. The composition for use according to any one of embodiments E25 to E33, wherein the application device (1) further comprises:
   f) an actuation unit (70) for the actuation of the application device such that the introduction/administration of the gaseous composition comprising carbon dioxide (24) into the nasal cavity of the subject is initiated.
E35. The composition for use according to embodiment E34, wherein the actuation unit (70) is adapted for manual actuation, preferably for manual actuation by the subject or other user.
E36. The composition for use according to any one of embodiments E25 to E35, wherein the pre-conditioning unit (20), specifically the pre-conditioning chamber (21) has an inlet (22) fluidically connected with the storage unit (10) and an outlet (23) fluidically connected with the application unit (30).
E37. The composition for use according to embodiment E36, wherein the inlet (22) of the pre-conditioning unit or chamber is fluidically connected to the storage unit (10) via a pressure reducer.
E38. The composition for use according to embodiment E35 or E37, wherein the outlet (23) of the pre-conditioning unit or chamber is fluidically connected to the application unit (30) via a valve (61), specifically via a metering valve and/or via a membrane.
E39. The composition for use according to any one of embodiments E25 to E38, wherein the application unit (30) has two plugs (36) adapted for introduction into both nostrils of the subject, preferably for simultaneous insertion into both nostrils of the subject.
E40. The composition for use according to any one of embodiments E25 to E39, wherein the application unit (30) comprises two application openings (34, 35) adapted to be introduced, specifically adapted to be simultaneously introduced into both nostrils of the subject.
E41. The composition for use according to any one of embodiments E25 to E40, wherein the application unit (30) is adapted for sealing both nostrils of the subject (when inserted into both nostrils of the subject).
E42. The composition for use according to any one of embodiments E25 to E41, wherein the application unit (30) comprises one or two plugs (36), preferably one or more flexible plugs, surrounding the application openings (34, 35) adapted for introduction and sealing closure of the nostrils of the subject.
E43. The composition for use according to any one of embodiments E25 to E42, wherein the applicator device (1) is a hand-held device that may be held and/or operated with one or two hands of a user, specifically the subject.
E44. An applicator device (1) for the intranasal administration of a pressurized gaseous composition comprising carbon dioxide (24) to a subject, preferably for the intranasal administration of the composition comprising carbon dioxide for use according to any one of embodiments E1 to E43,
   wherein the application device is adapted for the administration of the gaseous composition comprising carbon dioxide into the nasal cavity of the subject by intranasal administration via both nostrils of the subject, preferably via simultaneous administration via both nostrils of the subject.
E45. The applicator device (1) according to embodiment E44, wherein the applicator device comprises:
   a) a storage unit (10) for holding and/or providing the composition comprising carbon dioxide in pressurized form (12);
   b) a pre-conditioning unit (20) comprising a pre-conditioning chamber (21) fluidically connected with the storage unit (10) for transforming the composition comprising carbon dioxide in pressurized form (12) into a gaseous composition comprising carbon dioxide (24); and
   c) an application unit (30) fluidically connected with the pre-conditioning unit (20), specifically with the pre-conditioning chamber (21) for introducing the gaseous composition comprising carbon dioxide (24) into the nasal cavity of the subject via both nostrils of the subject; and optionally
   d) a housing (40) for holding the storage unit (10), the pre-conditioning unit (20), the application unit (30) and optionally further functional units of the applicator device (1).
E46. The applicator device (1) according to any one of embodiments E44 or E45, wherein the applicator device further comprises:
   e) a control assembly (50) for controlling at least one of the amount, pressure and flow rate at which the gaseous pharmaceutical composition (24) is administered to the nasal cavity of the patient.
E47. The applicator device (1) according to any one of embodiments E44 to E46, wherein the application device further comprises:
   f) an actuation unit (70) for the actuation of the administration device such that the introduction/administration of the gaseous composition comprising carbon dioxide (24) into the nasal cavity of the subject is initiated.
E48. A kit comprising:
   - a pressurized composition comprising carbon dioxide (12); and
   - an applicator device (1) adapted for the intranasal administration of the pressurized gaseous composition comprising carbon dioxide (12) to the nasal cavity of a subject via both nostrils of the subject, preferably an administration device according to any one of embodiments E44 to E47.
E49. The kit according to embodiment E48, wherein the storage unit (10) comprises a reservoir (11) and wherein the reservoir is provided in the form of a separate reservoir comprising a composition comprising carbon dioxide in pressurized form (12).
E50. The kit according to embodiments E48 or E49, further comprising instructions for use and/or assembly of the applicator device (1).
E51. A method for the preparation of a gaseous composition comprising carbon dioxide (24) (suitable for the administration into the nasal cavity of a subject), the method comprising the steps:
   - providing a composition comprising carbon dioxide in pressurized form (12), specifically in condensed form; and
   - at least partly evaporating the composition comprising carbon dioxide in pressurized form to provide a gaseous composition comprising carbon dioxide (in less pressurized form).
E52. The method according to embodiment E51, wherein the method comprises:
   - providing an applicator device (1) according to any one of embodiments E44 to E47.

## Claims

1. A pressurized composition comprising carbon dioxide (CO₂) for use
- in the treatment or prevention of a disorder or condition associated with headache, or a disorder or condition involving the trigeminal nervous system in a subject, or
- in the treatment or prevention of allergic rhinitis, rhinosinusitis, trigeminal neuralgia, post-traumatic headache, temporomandibular joint disorder, epilepsy or seizures in a subject,
wherein the composition comprising carbon dioxide is administered to the nasal cavity of the subject by intranasal administration in the form of a gaseous composition comprising carbon dioxide, and
wherein the gaseous composition comprising carbon dioxide is administered to the nasal cavity of the subject via both nostrils of the subject.

2. The composition for use according to claim 1, wherein the pressurized composition comprising carbon dioxide comprises carbon dioxide in a concentration of at least 50 mol-% or of at least 75 mol-% or of at least 90 mol-% (with regard to the total composition).

3. The composition for use according to claim 1 or 2, wherein the gaseous composition comprising carbon dioxide is administered in the form of a first dose administered within a treatment period of 10 min.

4. The composition for use according to any one of the preceding claims, wherein the gaseous composition is administered in the form of a sole dose, specifically in the form of a sole dose administered within the treatment period of 10 minutes.

5. The composition for use according to any one of the preceding claims, wherein during administration of the gaseous composition comprising carbon dioxide the subject does not inhale (breathe)/refrains from inhaling/breathing, specifically, wherein during administration of the gaseous composition comprising carbon dioxide the subject does not inhale (breathe)/refrains from inhaling/breathing through the nose.

6. The composition for use according to any one of the preceding claims, wherein the disorder or condition or condition associated with headache is migraine.

7. The composition for use according to any one of the preceding claims, wherein the gaseous composition is administered to the nasal cavity simultaneously via both nostrils of the subject.

8. The composition for use according to any one of the preceding claims, wherein the gaseous composition is administered to the nasal cavity of the subject at a temperature within range of from about 0 °C to about 18 °C or of from about 5 °C to about 15 °C.

9. The composition for use according to any one of the preceding claims, wherein the gaseous composition is administered to the nasal cavity of the subject at a pressure within the range of from about 1.5 bar to about 8 bar or of from about 4 bar to about 6 bar.

10. The composition for use according to any one of the preceding claims, wherein the gaseous composition is administered to the nasal cavity of the subject within a period of from about 5 s to about 30 s.

11. The composition for use according to any one of the preceding claims, wherein the gaseous composition is administered using an applicator device (1), preferably wherein the applicator device is adapted to generate an atmosphere enriched with carbon dioxide in the nasal cavity of the subject, specifically adapted to generate an atmosphere comprising a concentration of carbon dioxide within the range of from about 70 mol-% to about 99.9 mol-%.

12. The composition for use according to claim 11, wherein the applicator device (1) comprises:
a) a storage unit (10) for holding and/or providing the composition comprising carbon dioxide in pressurized form (12);
b) a pre-conditioning unit (20) comprising a pre-conditioning chamber (21) fluidically connected with the storage unit for transforming the composition comprising carbon dioxide in pressurized form into a gaseous composition comprising carbon dioxide (24); and
c) an application unit (30) fluidically connected with the pre-conditioning unit (20), specifically with the pre-conditioning chamber (21), for introducing the gaseous composition comprising carbon dioxide into the nasal cavity of the subject via both nostrils of the subject; and optionally
d) a housing (40) for holding the storage unit, the pre-conditioning unit, the application unit and optionally further functional units of the applicator device.

13. The composition for use according to claim 11 or 12, wherein the application device (1) further comprises:
e) a control unit (50) for controlling at least one of the amount, pressure, temperature and flow rate at which the gaseous composition comprising carbon dioxide (24) is administered to the nasal cavity of the subject.

14. An applicator device (1) for the intranasal administration of a gaseous composition comprising carbon dioxide (24) to a subject, preferably for the intranasal administration of the composition comprising carbon dioxide for use according to any one of claims 1 to 13,
wherein the application device is adapted for the administration of the gaseous composition comprising carbon dioxide into the nasal cavity of the subject by intranasal administration via both nostrils of the subject, preferably via simultaneous administration via both nostrils of the subject.

15. The applicator device (1) according to claim 14, wherein the applicator device is a hand-held device that may be held and/or operated with one or two hands of a user.
